# EUROPEAN PATENT SPECIFICATION

(11) **EP 3 864 002 B1**
(45) Date of publication and mention of the grant of the patent: **27.12.2023**
(21) Application number: 19790076.4
(22) Date of filing: 01.10.2019
(51) Int. Cl.: C07C 219/32, C07C 229/62

(54) **ADDITION-FRAGMENTATION AGENT WITH PENDENT AMINE GROUPS**
ADDITIONSFRAGMENTIERUNGSMITTEL MIT ANHÄNGENDEN AMINGRUPPEN
AGENT D'ADDITION-FRAGMENTATION À GROUPES AMINE PENDANTS

(30) Priority: 09.10.2018 US 201862742969 P
(43) Date of publication of application: 18.08.2021
(73) Proprietor: 3M Innovative Properties Company, Saint Paul, Minnesota 55133-3427 (US)
(72) Inventor: ABUELYAMAN, Ahmed S., Saint Paul, Minnesota 55133-3427 (US); GADDAM, Babu N., Saint Paul, Minnesota 55133-3427 (US); FALSAFI, Afshin, Saint Paul, Minnesota 55133-3427 (US); WU, Tianyu, Saint Paul, Minnesota 55133-3427 (US); WANG, Yizhong, Saint Paul, Minnesota 55133-3427 (US); CHRISTENSEN, Randilynn B., Saint Paul, Minnesota 55133-3427 (US); MOSER, William H., Saint Paul, Minnesota 55133-3427 (US)
(74) Representative: Mathys & Squire
(86) International application number: PCT/IB2019/058357
(87) International publication number: WO 2020/075007

(56) References cited:
- WO-A1-2014/151363
- WO-A1-2015/126657

## Description

### Background

The present disclosure provides novel addition-fragmentation agents (AFMs) for use in low-stress polymerizable compositions. Free-radical polymerization is typically accompanied by a reduction in volume as monomers are converted to polymer. The volumetric shrinkage produces stress in the cured composition, leading to a microcracks and deformation. Stress transferred to an interface between the cured composition and a substrate can cause failure in adhesion and can affect the durability of the cured composition.

The addition-fragmentation agents of this disclosure provide stress relief by including labile linkages that can cleave and reform during the polymerization process. Such cleavage may provide a mechanism to allow for network reorganization, relieve polymerization stress, and prevent the development of high stress regions. The instant addition-fragmentation agents may further provide stress relief by delaying the gel point, the point at which the polymerizable composition transitions from a viscous material to an elastic or viscoelastic solid. The longer the polymerizable mixture remains viscous, the more time available during which material flow can act to alleviate stress during the polymerization process.

The addition-fragmentation agents provide novel stress-reducing agents that have application in dental compositions, thin films, hardcoats, composites, adhesives, and other uses subject to stress reduction.

Patent application WO2015126657A1 discloses addition-fragmentation oligomers of the general formula Z-By-A-(B-A)x-B-A-By-Z, where the A monomers units are derived from a diester or diacid, the B monomer units are derived from a difunctional monomer having functional groups co-reactive with the acid or ester groups of the A monomer, x+y is zero to 60, Z comprises an ethylenically unsaturated, polymerizable group, A monomers comprise a 1-methylene-3,3-dimethylpropyl group, and at least one B monomer unit comprises a high refractive index group.

### Summary

The present disclosure provides addition-fragmentation agents according to the appended claims having the following functional groups: 1) a labile addition-fragmentation group that can cleave and reform to relieve strain, 2) an average of greater than 1, and preferably at least two tertiary amine groups, and 3) optionally at least one ethylenically unsaturated, polymerizable group.

In some embodiments this disclosure provides a polymerizable composition comprising the instant addition-fragmentation agent, at least one polymerizable monomer and a Type II photoinitiator. In some preferred embodiments the polymerizable monomer is a polymerizable dental resin.

Type II photoinitiators may be used to photoinitiate polymerization, but often slowly or inefficiently. Tertiary amines are often used as co-initiators to accelerate the rate of photopolymerization, whereby a co-initiator-centered radical initiates the polymerization. However, a problem associated with co-initiators, e.g. amines present in a radiation curable composition may arise when unreacted co-initiator remains in the cured composition. Hydrogen transfer from an amine co-initiator to a Type II photoinitiator is rarely quantitative. The unreacted co-initiator remains mobile in the cured composition and may adversely affect the physical properties of the cured composition or may diffuse out of the cured composition.

The instant disclosure overcomes problems associated with amine co-initiators by incorporating the tertiary amine in the molecule of the addition-fragmentation agent, thereby reducing issues of unreacted volatile amines in the polymer matrix. As the co-initiator groups are incorporated into the agent, the AFM structure is incorporated into the growing polymer matrix, allowing the AFM to form labile crosslinks and reduce polymerization stress and shrinkage.

In another embodiment, this disclosure provides a polymerizable composition comprising the instant addition-fragmentation agent, at least one polymerizable monomer, a transition metal that participates in a redox cycle, and a peroxy catalyst. The pendent tertiary amine groups serve as a reducing agent that works with the peroxy catalyst oxidant to produce a redox polymerizable composition.

This disclosure further provides a curable composition comprising the addition-fragmentation agent and one or more free-radically polymerizable monomers or oligomers. The addition-fragmentation agent provides a reduction in stress of the resultant polymers. The addition-fragmentation agents act as chain-transfer agents *via* an addition-fragmentation process whereby the addition-fragmentation linkages are labile during polymerization and continuously cleave and reform, providing a reduction in polymerization-based stress.

The addition-fragmentation agents may be added to polymerizable monomer mixtures to reduce the polymerization-induced stresses. This disclosure further provides a method of preparing the addition-fragmentation agents of formula I and of the formula according to the invention, as further disclosed herein.

This disclosure further provides a curable composition comprising the addition-fragmentation agent and one or more free-radically polymerizable monomers or oligomers, the addition-fragmentation agent providing a reduction in stress of the resultant polymers. The curable composition may be a dental composition comprising a dental resin and the addition-fragmentation agent.
As used herein:
"acryloyl" is used in a generic sense and mean not only derivatives of acrylic acid, but also amine, and alcohol derivatives, respectively;
"(meth)acryloyl" includes both acryloyl and methacryloyl groups; i.e. is inclusive of both esters and amides.
"curable" means that a coatable material can be transformed into a solid, substantially non-flowing material by means of free-radical polymerization, chemical cross linking, radiation crosslinking, or the like.
"alkyl" includes straight-chained, branched, and cycloalkyl groups and includes both unsubstituted and substituted alkyl groups. Unless otherwise indicated, the alkyl groups typically contain from 1 to 20 carbon atoms. Examples of "alkyl" as used herein include, but are not limited to, methyl, ethyl, n-propyl, n-butyl, n-pentyl, isobutyl, t-butyl, isopropyl, n-octyl, n-heptyl, ethylhexyl, cyclopentyl, cyclohexyl, cycloheptyl, adamantyl, and norbornyl, and the like. Unless otherwise noted, alkyl groups may be mono- or polyvalent, i.e. monvalent alkyl or polyvalent alkylene.
"heteroalkyl" includes both straight-chained, branched, and cyclic alkyl groups with one or more heteroatoms independently selected from S, O, and N with both unsubstituted and substituted alkyl groups. Unless otherwise indicated, the heteroalkyl groups typically contain from 1 to 20 carbon atoms. "Heteroalkyl" is a subset of "hydrocarbyl containing one or more S, N, O, P, or Si atoms" described below. Examples of "heteroalkyl" as used herein include, but are not limited to, methoxy, ethoxy, propoxy, 3,6-dioxaheptyl, 3-(trimethylsilyl)-propyl, 4-dimethylaminobutyl, and the like. Unless otherwise noted, heteroalkyl groups may be mono- or polyvalent, i.e. monovalent heteroalkyl or polyvalent heteroalkylene.
"aryl" is an aromatic group containing 5-18 ring atoms and can contain optional fused rings, which may be saturated, unsaturated, or aromatic. Examples of an aryl groups include phenyl, naphthyl, biphenyl, phenanthryl, and anthracyl. Heteroaryl is aryl containing 1-3 heteroatoms such as nitrogen, oxygen, or sulfur and can contain fused rings. Some examples of heteroaryl groups are pyridyl, furanyl, pyrrolyl, thienyl, thiazolyl, oxazolyl, imidazolyl, indolyl, benzofuranyl, and benzthiazolyl. Unless otherwise noted, aryl and heteroaryl groups may be mono- or polyvalent, i.e. monovalent aryl or polyvalent arylene.
"(hetero)hydrocarbyl" is inclusive of hydrocarbyl alkyl and aryl groups, and heterohydrocarbyl heteroalkyl and heteroaryl groups, the later comprising one or more catenary (in-chain) oxygen heteroatoms such as ether or amino groups. Heterohydrocarbyl may optionally contain one or more catenary (in-chain) functional groups including ester, amide, urea, urethane, and carbonate functional groups. Unless otherwise indicated, the non-polymeric (hetero)hydrocarbyl groups typically contain from 1 to 60 carbon atoms. Some examples of such heterohydrocarbyls as used herein include, but are not limited to, methoxy, ethoxy, propoxy, 4-diphenylaminobutyl, 2-(2'-phenoxyethoxy)ethyl, 3,6-dioxaheptyl, 3,6-dioxahexyl-6-phenyl, in addition to those described for "alkyl", "heteroalkyl", "aryl", and "heteroaryl" *supra.*

### Detailed Description

The present disclosure provides addition-fragmentation agents having the following functional groups: 1) a labile addition-fragmentation group that that can cleave and reform to relieve strain, and 2) an average of greater than one, preferably at least two tertiary amine groups. The labile addition-fragmentation group may be a 1-methylene-3,3-dimethylpropylene group.

It is generally disclosed an addition-fragmentation agent of the formula: 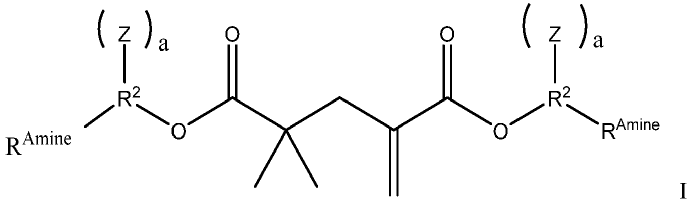 where
R^{Amine} comprises a pendent tertiary amine group;
R² linking group of valence a+2;
Z is an ethylenically unsaturated polymerizable group;
Subscript a is 0 or 1.

According to the invention, the addition-fragmentation agent is of the formula: 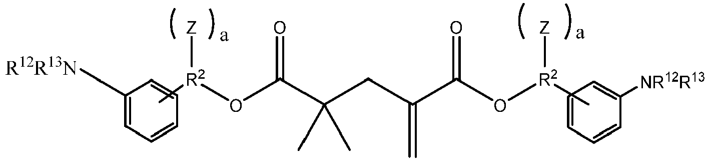 wherein
R² is a (hetero)hydocarbyl linking group of valence a+2
Z is an ethylenically unsaturated polymerizable group;
subscript a is 0 or 1
R¹² and R¹³ are independently selected from alkyl, aryl, alkaryl and aralkyl. R¹² and R¹³ may be taken together to form a heterocyclic ring, with that with the proviso that the group has at least one abstractable H atom alpha to the N atom.

With respect to Formula I, it will be appreciated that any of the depicted R² groups may have both an R^{Amine} and a polymerizable Z group and that the compound comprises greater than one and preferably at least two tertiary amine groups (R^{Amine} groups) and optionally at least one ethylenically unsaturated, polymerizable group.

The addition-fragmentation group can react into the polymeric system in which the labile group can be added to, fragment, and be added to again by a growing polymer chain to reduce the stress on the growing polymer or polymeric network.

The R^{Amine} groups may be any tertiary amine group having at least one abstractable hydrogen atom alpha to the N atom. The R^{Amine} may be of the formula:

-R¹⁴-R¹¹-N(R¹²)(R¹³)

where each -R¹¹, R¹², and R¹³ are selected from alkyl, aryl, alkaryl and aralkyl. R¹² and R¹³ may be taken together to form a heterocyclic ring, with that with the proviso that the group has at least one abstractable H atom alpha to the N atom,
-R¹⁴ is a covalent bond or (hetero)hydrocarbyl linking groups that links the tertiary amine group, -R¹¹-N(R¹²)(R¹³), to the R² group of Formula I.

Useful groups include dimethylaminopropyl, dimethylaminoethyl, dimethylaminobutyl, methylethylaminoethyl, dimethylaminobenzyl, diethylaminoethyl, diethylaminomethyl, phenylmethylaminoethyl, methylpropylaminomethyl tripropylamine, diisopropylaminopropyl, dibutylaminbutyl, diisobutylaminoethyl, dipentylaminomethyl, dipentylaminoethyl, dipentylaminopropyl dihexylaminopropyl, trioctylamine, triethanolamine, dibenzylaminoethyl, dibenzylaminomethyl, dibenzylaminopropyl, N-methylpiperazine and diinaphthylaminoethyl.

The agents of Formula I and of the invention may be prepared from (meth)acrylate dimers by substitution, displacement or condensation reactions. The starting (meth)acrylate dimers may be prepared by free radical addition of a (meth)acryloyl monomer in the presence of a free radical initiator and a cobalt (II) complex catalyst using the process of U.S. 4,547,323 (Carlson). Alternatively, the (meth)acryloyl dimers may be prepared using a cobalt chelate complex using the processes of U.S. 4,886,861 (Janowicz) or U.S. 5,324,879 (Hawthorne). In either process, the reaction mixture can contain a complex mixture of dimers, trimers, higher oligomers and polymers and the desired dimer can be separated from the mixture by distillation. As result, the agent of Formula I and of the invention often contain minor amounts of trimers and higher oligomers.

With reference to Formula I and to the formula of the invention, the requisite R^{amine} and the corresponding moiety in the formula of the invention and optional Z groups may be incorporated into the (meth)acryloyl dimer or trimer by means including addition, condensation, substitution and displacement reactions. In general, one or more of the acyl groups of the (meth)acryloyl dimer is provided with the requisite Ramine and the corresponding moiety in the formula of the invention and optional Z groups of Formula I and of the formula of the invention.

More specifically, a (meth)acryloyl compound of the formula: 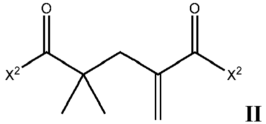
wherein X² (with the adjacent -CO-) comprises an electrophilic or nucleophilic functional group such as - an acid group, ester group or acid halide group, is reacted with a co-reactive compound of the formula:

   A²-R⁵*-(Y-R³)ₘ III
wherein
   A² is a functional group that is co-reactive with functional group X²,
   R⁵* is a single bond or a di- or trivalent (hetero)hydrocarbyl linking group that joins the Y group to reactive functional group A².
   R³ is alkyl, aryl, a coinitiators group, or an ethylenically unsaturated polymerizable group Z;
   Y is-O-. -S-, -O-CO-, O-CO-NH-, -N-CO-, or -NR⁴-, where R⁴ is H or C₁-C₄ alkyl;
   subscript m is 1-4;
   at least two of said R³ groups comprise tertiary amine groups R^{amine}.

More specifically, R⁵* is a single bond or a di- or trivalent linking group that joins an surface-binding group to co- reactive functional group A² and preferably contains up to 34, preferably up to 18, more preferably up to 10, carbon and, optionally, oxygen and nitrogen atoms, optional catenary ester, amide, urea, urethane and carbonate groups. When R⁵* is not a single bond, is may be selected from -O-. -S-, -N-R⁴-, -SO₂-, -PO₂-, -CO-, -OCO-, -N(R⁴)-CO-, -N(R⁴)-CO-O-, -N(R⁴)-CO-N(R⁴)-, -R⁶- and combinations thereof, such as -CO-O-R⁶-, -CO-N(R⁴)-R⁶-, and -R⁶-CO-O-R⁶-.
wherein each R⁴ is hydrogen, a C₁ to C₄ alkyl group, or aryl group, each R⁶ is an alkylene group having 1 to 6 carbon atoms, a 5- or 6-membered cycloalkylene group having 5 to 10 carbon atoms, or a divalent aromatic group having 6 to 16 carbon atoms.

It will be understood that reaction between the X² group of Formula II and the A² group of Formula III will form the R^{amine}-R²-O- moiety of Formula I, therefore may be defined as R^{amine}-R⁵-A^{2*}-X^{2*}-where A²*-X²*- is the bond formed between A² and X², as described *supra.* Therefore, the linkage may be defined as single bond or a divalent linking (hetero)hydrocarbyl group.

More particularly, the linkage is a single bond or a divalent linking group that joins a R^{amine} group to co- reactive functional group A and preferably contains up to 34, preferably up to 18, more preferably up to 10, carbon and, optionally, oxygen and nitrogen atoms, optional catenary ester, amide, urea, urethane and carbonate groups. When A²*-X²*- is not a single bond, it may be selected from -O-. -S-, - NR⁴-, -SO₂-, -PO₂-, -CO-, -OCO-, -R⁶- and combinations thereof, such as -N(R⁴)-CO-O-, -N(R⁴)-CON(R⁴)-, -CO-O-R⁶-, -CO-NR⁴-R⁶-, and -R⁶-CO-O-R⁶-, -O-R⁶-. -S-R⁶- -, -N(R⁴)-R⁶-, -SO₂-R⁶- , -PO₂-R⁶-, -CO-R⁶-, -OCO-R⁶-, -N(R⁴)-CO-R⁶-, NR⁴-R⁶-CO-O-, N(R⁴)-CO-N(R⁴)-, -R⁶-, with the proviso that Q'-Yₚ does not contain peroxidic linkages, i.e. O-O, N-O, S-O, N-N, N-S bonds, wherein each R⁴ is hydrogen, a C₁ to C₄ alkyl group, or aryl group, each R⁶ is an alkylene group having 1 to 6 carbon atoms, a 5- or 6-membered cycloalkylene group having 5 to 10 carbon atoms, or a divalent arylene group having 6 to 16 carbon atoms. Said alkylene groups are optionally substituted with one of more ether oxygen atoms or hydroxy gr5oups, alkoxy groups or aryloxy groups.

In some embodiments a compound of Formula II is reactive with a compound of Formula III, where A² comprises an epoxy or aziridine functional group. The reaction product has a hydroxyl group or amine group that may be further functionalized with additional R^{amine} groups, ethylenically unsaturated Z groups, or non-reactive groups such as alkyl or aryl groups.

In another embodiment, the starting material of Formula II may be reacted with a polyfunctional compound of the formula:

X⁵ₘ-R¹-X⁶ₘ,

wherein R¹ is a (hetero)alkyl group or a (hetero)aryl group and X⁵ comprises a functional group reactive with the nucleeophiol or electrophilic group (the X²) of the dimer to form an intermediate of the formula: 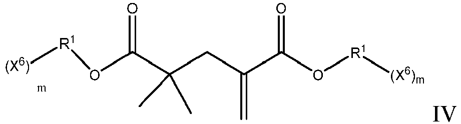 and X⁶ is a functional group capable of further functionalization, as described below.

A portion of the X⁶ groups of the intermediate may be reacted with a compound of the formula:

(Z)_{d}-X³, V

where Z comprises an ethylenically unsaturated polymerizable group, and X³ is a reactive functional group, reactive with the X⁶ groups of the intermediate, and d is at least 1.

A portion of the X⁶ groups of the intermediate may be reacted with a compound of the formula:

(R^{amine})_{d}-X³, VI

where R^{amine} comprises a coinitiator group, and X³ is a reactive functional group, reactive with the X⁶ groups of the intermediate.

With respect to the compound of Formulas IV-VI the requisite ethylenically unsaturated group and co-initiator group may be incorporated into the intermediate by means including addition, condensation, substitution and displacement reaction. The ethylenically unsaturated moiety, Z, may include, but is not limited to the following structures, including (meth)acryloyl, vinyl, styrenic and ethynyl, that are more fully described in reference to the preparation of the compounds below.

Generally, the intermediate IV is reacted with an unsaturated compound of the formula: 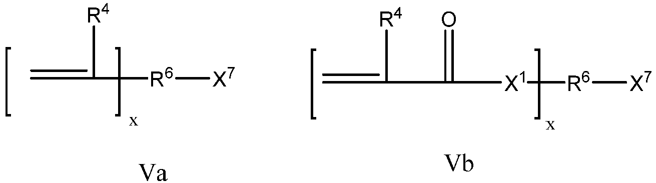 wherein
X⁷ is a functional group that is co-reactive with the functional group of the intermediate, R⁴ is hydrogen, a C₁ to C₄ alkyl group, R⁶ is a single bond or a divalent (hetero)hydrocarbyl linking group that joins the ethylenically unsaturated group to reactive functional group X¹ is -O- or -NR⁴-, where R⁴ is H or C₁-C₄ alkyl; and x is 1 or 2.

More particularly, the compound of Formula V may be of the formula:

Y¹-R¹³-O-CO-CR¹⁴=CH₂, VIIa

where Y¹ is an electrophilic functional group reactive with nucleophilic X² groups, R¹³ is a (hetero)hydrocarbyl group, preferably alkylene, R¹⁴ is H or C₁-C₄ alkyl,
or of the formula

   Y²-R¹³-O-CO-CR¹⁴=CH₂, VIIb
where Y² is a nucleophilic functional group reactive with electrophilic X² groups, R¹³ is (hetero)hydrocarbyl group, preferably alkylene, and R¹⁴ is H or C₁-C₄ alkyl.

In reference to Formulas Va, b, particularly useful groups include H₂C=C(CH₃)C(O)-O-CH₂-CH(OH)-CH₂-O-, H₂C=C(CH₃)C(O)-O-CH₂-CH(O-(O)C(CH₃)=CH₂)-CH₂-O-, H₂C=C(CH₃)C(O)-O-CH(CH₂OPh)-CH₂-O-, H₂C=C(CH₃)C(O)-O-CH₂CH₂-N(H)-C(O)-O-CH(CH₂OPh)-CH₂-O-., H₂C=C(CH₃)C(O)-O-CH₂-CH(O-(O)C-N(H)-CH₂CH₂-O-(O)C(CH₃)C=CH₂)-CH₂-O-, H₂C=C(H)C(O)-O-(CH₂)₄-O-CH₂-CH(OH)-CH₂-O-, H₂C=C(CH₃)C(O)-O-CH₂-CH(O-(O)C-N(H)-CH₂CH₂-O-(O)C(CH₃)C=CH₂)-CH₂-O-, CH₃-(CH₂)₇ -CH(O-(O)C-N(H)-CH₂CH₂-O-(O)C(CH₃)C=CH₂)-CH₂-O-, H₂C=C(H)C(O)-O-(CH₂)₄-O-CH₂-CH(-O-(O)C(H)=CH₂)-CH₂-O- and H₂C=C(H)C(O)-O-CH₂-CH(OH)-CH₂-O-. H₂C=C(H)C(O)-O-(CH₂)₄-O-CH₂-CH(-O-(O)C(H)=CH₂)-CH₂-O-, and CH₃-(CH₂)₇ -CH(O-(O)C-N(H)-CH₂CH₂-O-(O)C(CH₃)C=CH₂)-CH₂-O- .

As described *supra,* a portion of the X² groups of intermediate IV may be reacted with a compound of the formula: (R^{RI})_{d}-X³, VI
where R^{amine} comprises a tertiary amine group, and X³ is a reactive functional group, reactive with the X² groups of the intermediate. As described for the Z group, the requisite R^{amine} group may be incorporated into the intermediate by means including addition, condensation, substitution and displacement reaction.

More particularly, the compound of Formula VI may be of the formula:

Y¹-(R³)_{q}-X⁴-R^{amine}, VIa

where Y¹ is an electrophilic functional group reactive with nucleophilic X² groups, R³ is a (hetero)hydrocarbyl group, preferably alkylene, q is 0 or 1, X⁴ is selected from a covalent bond or a divalent linking group including -O-, -O-CO-, -O-CO-NH-, -S-, -NH-, -NH-CO-, -NH-CO-NH, -NH-CO-O-, -O-CO-NH
and R^{amine} is a coinitiator group;
or of the formula

   Y²-(R³)_{q}- X⁴-R^{RI*}, VIb
where Y² is an nucleophilic functional group reactive with electrophilic X² groups, R³ is (hetero)hydrocarbyl group, preferably alkylene, q is 0 or 1, X⁴ is selected from a covalent bond or a divalent linking group including -O-, -O-CO-, -O-CO-NH-, -S-, -NH-, -NH-CO-, -NH-CO-NH, -NH-CO-O-, -O-CO-NH
and R^{amine} comprises a tertiary amine group.

Useful Y¹ and Y² groups of compounds of formulas VI a,b include hydroxyl, amino, oxazolinyl, oxazolonyl, acetyl, acetonyl, carboxyl, isocyanato, epoxy, aziridinyl, acyl halide, halide and cyclic anhydride groups. Where the reactive functional group X² is an isocyanato functional group, the co-reactive functional Y² group preferably comprises a amino or hydroxyl group. Where the pendent reactive functional group X² comprises a hydroxyl group, the co-reactive functional group Y¹ preferably comprises a carboxyl, ester, acyl halide, isocyanato, epoxy, anhydride, azlactonyl or oxazolinyl group. Where the pendent reactive functional group comprises a X² carboxyl group, the co-reactive functional Y² group preferably comprises a hydroxyl, amino, epoxy, isocyanate, or oxazolinyl group.

In some preferred embodiments, the intermediate of Formula IV a glycidyl methacrylate dimer, where the epoxy groups may be functionalized as follows:

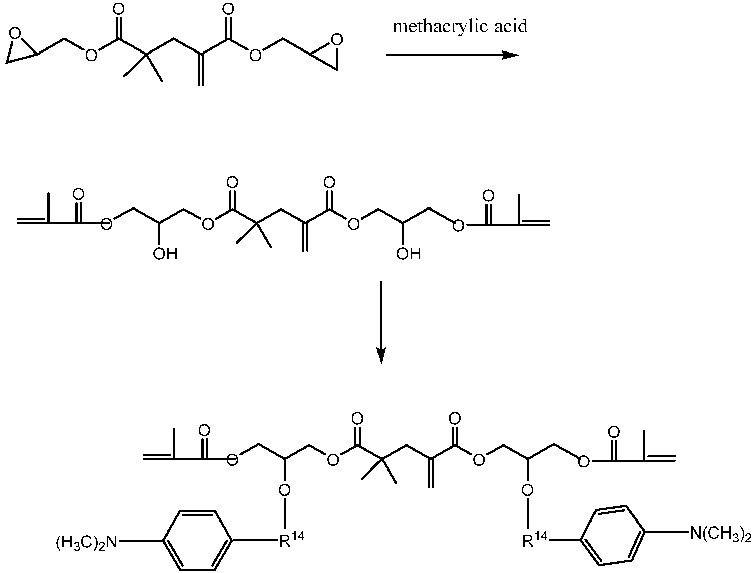

Alternatively, the intermediate of Formula IV is the dimer of methacrylic acid, which is first functionalized with glycidyl methacrylate (gma) to produce the methacrylate-functional intermediate, the hydroxyl of which is then further functionalized to provide the R^{amine} group or the corresponding moiety in the formula of the invention. It will be understood that different isomers from those depicted may result from the ring-opening. The illustrated products, having hydroxyl groups, may then be provided with the R^{amine} group or the corresponding moiety in the formula of the invention by reaction with a compound of the formula IIb: an amine or
A²-R⁵*-R^{amine}. Alternatively, one may start with an aziridine compound to produce the corresponding amine functional intermediate.

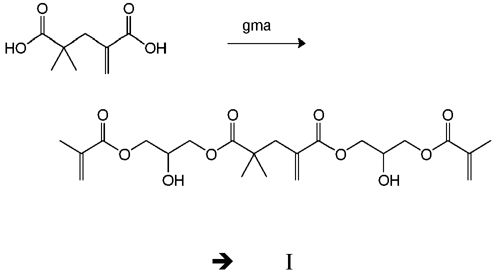

The present disclosure further provides a polymerizable composition comprising the addition-fragmentation agent of the invention , and at least one polymerizable monomer, such as (meth)acryloyl monomers, including acrylate esters, amides, and acids to produce (meth)acrylate homo- and copolymers. Generally, the addition-fragmentation agent of the invention is used in amounts of 0.1 to 10 parts by weight, preferably 0.1 to 5 parts by weight, based on 100 parts by weight of total monomer.

The (meth)acrylate ester monomer useful in preparing the (meth)acrylate polymer is a monomeric (meth)acrylic ester of a non-tertiary alcohol, which alcohol contains from 1 to 14 carbon atoms and preferably an average of from 4 to 12 carbon atoms.

Examples of monomers suitable for use as the (meth)acrylate ester monomer include the esters of either acrylic acid or methacrylic acid with non-tertiary alcohols such as ethanol, 1-propanol, 2-propanol, 1-butanol, 2-butanol, 1-pentanol, 2-pentanol, 3-pentanol, 2-methyl-1-butanol, 3-methyl-1-butanol, 1-hexanol, 2-hexanol, 2-methyl-1-pentanol, 3-methyl-1-pentanol, 2-ethyl-1-butanol, 3,5,5-trimethyl-1-hexanol, 3-heptanol, 1-octanol, 2-octanol, isooctylalcohol, 2-ethyl-1-hexanol, 1-decanol, 2-propylheptanol, 1-dodecanol, 1-tridecanol, 1-tetradecanol, citronellol, dihydrocitronellol, and the like. In some embodiments, the preferred (meth)acrylate ester monomer is the ester of (meth)acrylic acid with butyl alcohol or isooctyl alcohol, or a combination thereof, although combinations of two or more different (meth)acrylate ester monomer are suitable. In some embodiments, the preferred (meth)acrylate ester monomer is the ester of (meth)acrylic acid with an alcohol derived from a renewable source, such as 2-octanol, citronellol, or dihydrocitronellol.

In some embodiments it is desirable for the (meth)acrylic acid ester monomer to include a high T_{g} monomer. The homopolymers of these high T_{g} monomers have a T_{g} of at least 25°C, and preferably at least 50°C. Examples of suitable monomers useful in the present invention include, but are not limited to, t-butyl acrylate, methyl methacrylate, ethyl methacrylate, isopropyl methacrylate, n-butyl methacrylate, isobutyl methacrylate, s-butyl methacrylate, t-butyl methacrylate, stearyl methacrylate, phenyl methacrylate, cyclohexyl methacrylate, isobornyl acrylate, isobornyl methacrylate, benzyl methacrylate, 3,3,5 trimethylcyclohexyl acrylate, cyclohexyl acrylate, N-octyl acrylamide, and propyl methacrylate or combinations.

The (meth)acrylate ester monomer is present in an amount of up to 100 parts by weight, preferably 85 to 99.5 parts by weight based on 100 parts total monomer content used to prepare the polymer, exclusive of the amount of multifunctional (meth)acrylates. Preferably (meth)acrylate ester monomer is present in an amount of 90 to 95 parts by weight based on 100 parts total monomer content. When high T_{g} monomers are included, the copolymer may include up to 50 parts by weight, preferably up to 20 parts by weight of the (meth)acrylate ester monomer component.

The polymer may further comprise an acid functional monomer, where the acid functional group may be an acid *per se,* such as a carboxylic acid, or a portion may be a salt thereof, such as an alkali metal carboxylate. Useful acid functional monomers include, but are not limited to, those selected from ethylenically unsaturated carboxylic acids, ethylenically unsaturated sulfonic acids, ethylenically unsaturated phosphonic or phosphoric acids, and mixtures thereof. Examples of such compounds include those selected from acrylic acid, methacrylic acid, itaconic acid, fumaric acid, crotonic acid, citraconic acid, maleic acid, oleic acid, β-carboxyethyl (meth)acrylate, 2-sulfoethyl methacrylate, styrene sulfonic acid, 2-acrylamido-2-methylpropanesulfonic acid, vinylphosphonic acid, and mixtures thereof.

Due to their availability, acid functional monomers of the acid functional copolymer are generally selected from ethylenically unsaturated carboxylic acids, i.e. (meth)acrylic acids. When even stronger acids are desired, acidic monomers include the ethylenically unsaturated sulfonic acids and ethylenically unsaturated phosphonic acids. The acid functional monomer is generally used in amounts of 0.5 to 15 parts by weight, preferably 1 to 15 parts by weight, most preferably 5 to 10 parts by weight, based on 100 parts by weight total monomer.

The polymer may further comprise a polar monomer. The polar monomers useful in preparing the copolymer are both somewhat oil soluble and water soluble, resulting in a distribution of the polar monomer between the aqueous and oil phases in an emulsion polymerization. As used herein the term "polar monomers" are exclusive of acid functional monomers.

Representative examples of suitable polar monomers include but are not limited to 2-hydroxyethyl (meth)acrylate; N-vinylpyrrolidone; N-vinylcaprolactam; acrylamide; mono- or di-N-alkyl substituted acrylamide; t-butyl acrylamide; dimethylaminoethyl acrylamide; N-octyl acrylamide; poly(alkoxyalkyl) (meth)acrylates including 2-(2-ethoxyethoxy)ethyl (meth)acrylate, 2-ethoxyethyl (meth)acrylate, 2-methoxyethoxyethyl (meth)acrylate, 2-methoxyethyl methacrylate, polyethylene glycol mono(meth)acrylates; alkyl vinyl ethers, including vinyl methyl ether; and mixtures thereof. Preferred polar monomers include those selected from the group consisting of 2-hydroxyethyl (meth)acrylate and N-vinylpyrrolidinone. The polar monomer may be present in amounts of 0 to 10 parts by weight, preferably 0.5 to 5 parts by weight, based on 100 parts by weight total monomer.

The polymer may further comprise a vinyl monomer. When used, vinyl monomers useful in the (meth)acrylate polymer include vinyl esters (e.g., vinyl acetate and vinyl propionate), styrene, substituted styrene (e.g., α-methyl styrene), vinyl halide, and mixtures thereof. As used herein vinyl monomers are exclusive of acid functional monomers, acrylate ester monomers and polar monomers. Such vinyl monomers are generally used at 0 to 5 parts by weight, preferably 1 to 5 parts by weight, based on 100 parts by weight total monomer.

A multifunctional (meth)acrylate may be incorporated into the blend of polymerizable monomers (in addition to the addition-fragmentation agent). Examples of useful multifunctional (meth)acrylates include, but are not limited to, di(meth)acrylates, tri(meth)acrylates, and tetra(meth)acrylates, such as 1,6-hexanediol di(meth)acrylate, polyethylene glycol) di(meth)acrylates, polybutadiene di(meth)acrylate, polyurethane di(meth)acrylates, and propoxylated glycerin tri(meth)acrylate, and mixtures thereof. The amount and identity of multifunctional (meth)acrylate is tailored depending upon application of the adhesive composition, for example, adhesives, hardcoats or dental resins. Typically, the multifunctional (meth)acrylate is present in amounts up to 100 parts based on the weight of remaining polymerizable composition. In some embodiments the multifunctional (meth)acrylate is used in amounts of 50 parts by weight or more, based on the weight of remaining polymerizable composition. In some embodiments, the crosslinker may be present in amounts from 0.01 to 5 parts, preferably 0.05 to 1 parts, based on 100 parts total monomers of the adhesive composition for adhesive applications, and greater amounts for hardcoats or dental resins, as described herein.

In such embodiments, the copolymer may comprise:
i. up to 100 parts by weight, preferably 85 to 99.5 parts by weight of an (meth)acrylic acid ester;
ii. 0 to 15 parts by weight, preferably 0.5 to 15 parts by weight of an acid functional ethylenically unsaturated monomer;
iii. 0 to 15 parts by weight of a non-acid functional, ethylenically unsaturated polar monomer;
iv. 0 to 5 parts vinyl monomer;
v. 0 to 100 parts of a multifunctional (meth)acrylate, relative to i-iv;
vi. 0 to 5 parts of a polymerizable photoinitiator.

based on 100 parts by weight total monomer and
0.1 to 10 parts of the addition-fragmentation agent, relative to 100 parts total monomer.

In some embodiments the multifunctional (meth)acrylate may be a a reactive oligomer having pendent polymerizable groups comprising:
a) greater than 50 parts by weight, preferably greater than 75 parts by weight, most preferably greater than 80 parts by weight of (meth)acrylate ester monomer units;
b) 1 to 10 parts by weight, preferably 1 to 5 parts by weight, most preferably 1 to 3 parts by weight, of monomer units having a pendent, free-radically polymerizable functional group,
c) 0 to 20 parts by weight of other polar monomer units, wherein the sum of the monomer units is 100 parts by weight.

The reactive oligomer may be represented by the formula:

-[M^{Unsatd}]ₒ[M^{ester}]ₚ[M^{polar}]_{q}-, VII

where
[M^{Unsatd}] represents monomer units having a pendent, free-radically polymerizable functional groups and subscript "o" is the parts be weight thereof;
[M^{ester}] represents (meth)acrylate ester monomer units and subscript "p" represents the parts by weight thereof; and
[M^{polar}] represents polar monomer units and subscript "q" represents the parts by weight thereof.

The reactive oligomers (VII) of the composition comprise one or more pendent groups that include free-radically polymerizable unsaturation, including (meth)acryloyl, (meth)acryloxy, propargyl, vinyl, allyl, acetylenyl and (meth)acrylamide. That is, the monomer units [M^{Unsatd}] contain such polymerizable groups.

The polymerizable reactive oligomer component may further comprise a diluent monomer. The (meth)acrylate-functional diluents, also referred to herein as "reactive diluents", are relatively low molecular weight mono- or di-functional, non-aromatic, (meth)acrylate monomers. These relatively low molecular weight reactive diluents are advantageously of a relatively low viscosity, e.g., less than about 30mPas (30 centipoise) at 25°C. Di-functional, non-aromatic (meth)acrylates are generally preferred over mono- functional non-aromatic (meth)acrylates because di-functional non-aromatic (meth)acrylates allow for quicker cure time. Preferred reactive diluents include 1,6-hexanediol di(meth)acrylate (HDDA from UCB Radcure, Inc. of Smyrna, Georgia), tripropylene glycol di(meth)acrylate, isobornyl (meth)acrylate (1130A, Radcure), 2(2-ethoxyethoxy) ethyl (meth)acrylate (sold under the trade name Sartomer 256 from SARTOMER Company, Inc. of Exton, Pennsylvania), n-vinyl formamide (Sartomer 497), tetrahydrofurfuryl (meth)acrylate (Sartomer 285), polyethylene glycol di(meth)acrylate (Sartomer 344), tripropylene glycol di(meth)acrylate (Radcure), neopentyl glycol dialkoxy di(meth)acrylate, polyethyleneglycol di(meth)acrylate, and mixtures thereof.

In some embodiments the polymerizable composition may comprise:
20-80 parts by weight of multifunctional (meth)acrylate monomers and/or multifunctional (meth)acrylate reactive oligomers,
0 to parts by weight range of (meth)acrylate diluent,
20 to 75 wt.% of silica (*per se,* whether or not functionalized), and
from about 0.1 weight percent to about 5.0 weight percent of the redox initiator system, based on the 100 parts by weight of the polymerizable components of the polymerizable composition.

The present disclosure further provides curable dental compositions comprising the addition-fragmentation agent of the invention. Although various curable dental compositions have been described, industry would find advantage in compositions having improved properties such as reduced stress deflection and/or reduced shrinkage while maintaining sufficient mechanical properties and depth of cure.

As used herein, "dental composition" refers to a material, optionally comprising filler, capable of adhering or being bonded to an oral surface. A curable dental composition can be used to bond a dental article to a tooth structure, form a coating (e.g., a sealant or varnish) on a tooth surface, be used as a restorative that is placed directly into the mouth and cured in-situ, or alternatively be used to fabricate a prosthesis outside the mouth that is subsequently adhered within the mouth.

Curable dental compositions include, for example, adhesives (e.g., dental and/or orthodontic adhesives), cements (e.g., resin-modified glass ionomer cements, and/or orthodontic cements), primers (e.g., orthodontic primers), liners (applied to the base of a cavity to reduce tooth sensitivity), coatings such as sealants (e.g., pit and fissure), and varnishes; and resin restoratives (also referred to as direct composites) such as dental fillings, as well as crowns, bridges, and articles for dental implants. Highly filled dental compositions are also used for mill blanks, from which a crown may be milled. A composite is a highly filled paste designed to be suitable for filling substantial defects in tooth structure. Dental cements are somewhat less filled and less viscous materials than composites, and typically act as a bonding agent for additional materials, such as inlays, onlays and the like, or act as the filling material itself if applied and cured in layers. Dental cements are also used for permanently bonding dental restorations such as a crown or bridge to a tooth surface or an implant abutment.

The total amount of addition-fragmentation agent(s) in the polymerizable resin portion of the unfilled curable dental composition is typically no greater than 15 wt.%. As the concentration of the addition-fragmentation monomer increases, the stress deflection and Watts Shrinkage typically decrease. However, when the amount of addition-fragmentation agent exceeds an optimal amount, mechanical properties such as Diametral tensile strength and/or Barcol hardness, or depth of cure may be insufficient.

The polymerizable resin portion of the curable dental composition described herein comprises at least 0.1 wt.%, of addition-fragmentation agent(s). Generally, the amount of addition-fragmentation agent is from about 0.5 to 10 wt.% of the polymerizable portion of the unfilled dental composition.

Materials with high polymerization stress upon curing generate strain in the tooth structure. One clinical consequence of such stress can be a decrease in the longevity of the restoration. The stress present in the composite passes through the adhesive interface to the tooth structure generating cuspal deflection and cracks in the surrounding dentin and enamel which can lead to postoperative sensitivity as described in R. R. Cara et al, Particulate Science and Technology 28; 191-206 (2010). Preferred (e.g. filled) dental compositions (useful for restorations such as fillings and crowns) described herein typically exhibit a stress deflection of no greater than 2.0, or 1.8, or 1.6, or 1.4, or 1.2 or 1.0 or 0.8 or 0.6 microns.

The curable compositions described herein further comprise at least one ethylenically unsaturated resin monomer or oligomer in combination with the addition-fragmentation agent. In some embodiments, such as primers, the ethylenically unsaturated monomer may be monofunctional, having a single (e.g. terminal) ethylenically unsaturated group. In other embodiments, such as dental restorations the ethylenically unsaturated monomer is multifunctional. The phrase "multifunctional ethylenically unsaturated" means that the monomers each comprise at least two ethylenically unsaturated (e.g. free radically) polymerizable groups, such as (meth)acrylate groups.

The amount of curable resin in the dental composition is a function of the desired end use (adhesives, cements, restoratives, etc.) and can be expressed with respect to the (i.e. unfilled) polymerizable resin portion of the dental composition. For favored embodiments, wherein the composition further comprises filler, the concentration of monomer can also be expressed with respect to the total (i.e. filled) composition. When the composition is free of filler, the polymerizable resin portion is the same as the total composition.

In favored embodiments, such ethylenically unsaturated groups of the curable dental resin includes (meth)acryloyl such as (meth)acrylamide and (meth)acrylate. Other ethylenically unsaturated polymerizable groups include vinyl and vinyl ethers. The ethylenically unsaturated terminal polymerizable group(s) is preferably a (meth)acrylate group, particularly for compositions that are hardened by exposure to actinic (e.g. UV and visible) radiation. Further, methacrylate functionality is typically preferred over the acrylate functionality in curable dental compositions. The ethylenically unsaturated monomer may comprise various ethylenically unsaturated monomers, as known in the art, for use in dental compositions.

In favored embodiments, the (e.g. dental) composition comprises one or more dental resins having a low volume shrinkage monomer. Preferred (e.g. filled) curable dental compositions (useful for restorations such as fillings and crowns) comprise one or more low volume shrinkage resins such that the composition exhibits a Watts Shrinkage of less than about 2%, preferably no greater than 1.80%, more no greater than 1.60%. In favored embodiments, the Watts Shrinkage is no greater than 1.50%, or no greater than 1.40%, or no greater than 1.30%, and in some embodiments no greater than 1.25%, or no greater than 1.20%, or no greater than 1.15%, or no greater than 1.10%.

Preferred low volume shrinkage monomers include isocyanurate resins, such as described in U.S.S.N. 2013/0012614 (Abuelyaman et al.) ; tricyclodecane resins, such as described in US 8710113 (Eckert et al.); polymerizable resins having at least one cyclic allylic sulfide moiety such as described in U.S. 7,888,400 (Abuelyaman et al.); methylene dithiepane silane resins as described in US 6,794,520 (Moszner et al.); and di-, tri, and/or tetra-(meth)acryloyl-containing resins such as described in U.S. 2010/021869 (Abuelyaman et al.).

In favored embodiments, the majority of the (e.g. unfilled) polymerizable resin composition comprises one or more low volume shrinkage monomers ("Low shrinkage monomers"). For example, at least 50%, 60%, 70%, 80%, 90% or more of the (e.g. unfilled) polymerizable resin may comprise low volume shrinkage monomer(s).

In one embodiment, the dental composition comprises at least one isocyanurate resin. The isocyanurate resin comprises a trivalent isocyanuric acid ring as an isocyanurate core structure and at least two ethylenically unsaturated (e.g. free radically) polymerizable groups bonded to at least two of the nitrogen atoms of the isocyanurate core structure via a (e.g. divalent) linking group. The linking group is the entire chain of atoms between the nitrogen atom of the isocyanurate core structure and the terminal ethylenically unsaturated group. The ethylenically unsaturated (e.g. free radically) polymerizable groups are generally bonded to the core or backbone unit via a (e.g. divalent) linking group.

In another embodiment, the dental composition comprises at least one tricyclodecane resin. The tricyclodecane resin may comprise a single monomer or a blend of two or more tricyclodecane resins. The concentration of multifunctional ethylenically unsaturated tricyclodecane monomer in the (i.e. unfilled) polymerizable resin portion or filled hardenable (i.e. polymerizable) composition can be the same as just described for the multifunctional ethylenically unsaturated isocyanurate monomer.

In some embodiments, the curable dental composition comprises a polymerizable resin having at least one cyclic allylic sulfide moiety with at least one (meth)acryloyl moiety.

The cyclic allylic sulfide moiety typically comprises at least one 7- or 8-membered ring that has two heteroatoms in the ring, one of which is sulfur. Most typically both of the heteroatoms are sulfur, which may optionally be present as part of an SO, SO₂, or S-S moiety. In other embodiments, the ring may comprise a sulfur atom plus a second, different heteroatom in the ring, such as oxygen or nitrogen. In addition, the cyclic allylic moiety may comprise multiple ring structures, i.e. may have two or more cyclic allylic sulfide moieties. The (meth)acryloyl moiety is preferably a (meth)acryloyloxy (i.e. a (meth)acrylate moiety) or a (meth)acryloylamino (i.e., a (meth)acrylamide moiety).

In another embodiment, the low shrinkage dental resin may be selected from methylene dithiepane silane resins described in U.S. 6,794,520 (Moszner et al.).

Particularly for dental restoration compositions, the ethylenically unsaturated resins generally have a refractive index of at least 1.50. In some embodiments, the refractive index is at least 1.51, 1.52, 1.53, or greater. The inclusion of sulfur atoms and/or the present of one or more aromatic moieties can raise the refractive index (relative to the same molecular weight resin lacking such substituents).

In some embodiments, the (unfilled) polymerizable resin may comprise solely one or more low shrink resins in combination with the addition fragmentation agent(s). In other embodiments, the (unfilled) polymerizable resin comprises a small concentration of other monomer(s). By "other" is it meant an ethylenically unsaturated monomer such as a (meth)acrylate monomer that is not a low volume shrinkage monomer.

The concentration of such other monomer(s) is typically no greater than 20 wt.%, 19 wt.%, 18 wt.%, 17 wt.%, 16 wt.%, or 15 wt.% of the (unfilled) polymerizable resin portion. The concentration of such other monomers is typically no greater than 5 wt.%, 4 wt.%, 3 wt.%, or 2 wt.% of the filled polymerizable dental composition.

In some embodiments, the "other monomers" of the dental composition comprise a low viscosity reactive (i.e. polymerizable) diluent. Reactive diluents typically have a viscosity of no greater than 300 Pa*s and preferably no greater than 100 Pa*s, or 50 Pa*s, or 10 Pa*s. In some embodiments, the reactive diluent has a viscosity no greater than 1 or 0.5 Pa*s. Reactive diluents are typically relatively low in molecular weight, having a molecular weight less than 600 g/mole, or 550 g/mol, or 500 g/mole. Reactive diluents typically comprise one or two ethylenically unsaturated groups such as in the case of mono(meth)acrylate or di(meth)acrylate monomers.

In some embodiments, the reactive diluent is an isocyanurate or tricyclodecane monomer. Tricyclodecane reactive diluent may have the same generally structure as previously described. In favored embodiments, the tricyclodecane reactive diluent
comprises one or two spacer unit(s) being connected to the backbone unit (U) via an ether linkage; such as described in U.S. 9012531 (Eckert et al.).

Although the inclusion of an addition fragmentation agent in a low volume shrinkage composition typically provides the lowest stress and/or lowest shrinkage, the addition fragmentation agents described herein can also reduce the stress of dental composition comprising conventional hardenable (meth)acrylate monomers, such as ethoxylated bisphenol A dimethacrylate (BisEMA6), 2-hydroxyethyl methacrylate (HEMA), bisphenol A diglycidyl dimethacrylate (bisGMA), urethane dimethacrylate (UDMA), triethlyene glycol dimethacrylate (TEGDMA), glycerol dimethacrylate (GDMA), ethyleneglycol dimethacrylate, neopentylglycol dimethacrylate (NPGDMA), and polyethyleneglycol dimethacrylate (PEGDMA).

The curable component of the curable dental composition can include a wide variety of "other" ethylenically unsaturated compounds (with or without acid functionality), epoxy-functional (meth)acrylate resins, vinyl ethers, and the like.

The (e.g., photopolymerizable) dental compositions may include free radically polymerizable monomers, agents, and polymers having one or more ethylenically unsaturated groups. Suitable compounds contain at least one ethylenically unsaturated bond and are capable of undergoing addition polymerization. Examples of useful ethylenically unsaturated compounds include acrylic acid esters, methacrylic acid esters, hydroxy-functional acrylic acid esters, hydroxy-functional methacrylic acid esters, and combinations thereof.

The dental compositions described herein may include one or more curable components in the form of ethylenically unsaturated compounds with acid functionality as an example of an "other" monomer. When present, the polymerizable component optionally comprises an ethylenically unsaturated compound with acid functionality. Preferably, the acid functionality includes an oxyacid (i.e., an oxygen-containing acid) of carbon, sulfur, phosphorous, or boron. Such acid-functional "other" monomers contribute to the self-adhesion or self-etching of the dental compositions as described in U.S. 2005/017966 (Falsafi et al.).

As used herein, ethylenically unsaturated compounds with acid functionality is meant to include monomers, oligomers, and polymers having ethylenic unsaturation and acid and/or acid-precursor functionality. Acid-precursor functionalities include, for example, anhydrides, acid halides, and pyrophosphates. The acid functionality can include carboxylic acid functionality, phosphoric acid functionality, phosphonic acid functionality, sulfonic acid functionality, or combinations thereof.

Ethylenically unsaturated compounds with acid functionality include, for example, α,β-unsaturated acidic compounds such as glycerol phosphate mono(meth)acrylates, glycerol phosphate di(meth)acrylates, hydroxyethyl (meth)acrylate (e.g., HEMA) phosphates, bis((meth)acryloxyethyl) phosphate, bis((meth)acryloxypropyl) phosphate, bis((meth)acryloxy)propyloxy phosphate, (meth)acryloxyhexyl phosphate, bis((meth)acryloxyhexyl) phosphate, (meth)acryloxyoctyl phosphate, bis((meth)acryloxyoctyl) phosphate, (meth)acryloxydecyl phosphate, bis((meth)acryloxydecyl) phosphate, caprolactone methacrylate phosphate, citric acid di- or tri-methacrylates, poly(meth)acrylated oligomaleic acid, poly(meth)acrylated polymaleic acid, poly(meth)acrylated poly(meth)acrylic acid, poly(meth)acrylated polycarboxyl-polyphosphonic acid, poly(meth)acrylated polychlorophosphoric acid, poly(meth)acrylated polysulfonate, poly(meth)acrylated polyboric acid, and the like, may be used as components. Also monomers, oligomers, and polymers of unsaturated carbonic acids such as (meth)acrylic acids, itaconic acid, aromatic (meth)acrylated acids (e.g., methacrylated trimellitic acids), and anhydrides thereof can be used.

The dental compositions can include an ethylenically unsaturated compound with acid functionality having at least one P-OH moiety. Such compositions are self-adhesive and are non-aqueous. For example, such compositions can include: a first compound including at least one (meth)acryloxy group and at least one -O-P(O)(OH)ₓ group, wherein x=1 or 2, and wherein the at least one -O-P(O)(OH)ₓ group and the at least one (meth)acryloxy group are linked together by a C₁-C₄ hydrocarbon group; a second compound including at least one (meth)acryloxy group and at least one -O-P(O)(OH)ₓ group, wherein x=1 or 2, and wherein the at least one -O-P(O)(OH)ₓ group and the at least one (meth)acryloxy group are linked together by a C₅-C₁₂ hydrocarbon group; an ethylenically unsaturated compound without acid functionality; an initiator system; and a filler.

The curable dental compositions can include at least 1 wt.%, at least 3 wt.%, or at least 5 wt.% ethylenically unsaturated compounds with acid functionality, based on the total weight of the unfilled composition. The compositions can include at most 80 wt.%, at most 70 wt.%, or at most 60 wt.% ethylenically unsaturated compounds with acid functionality.

Dental compositions suitable for use as dental adhesives can optionally also include filler in an amount of at least 1 wt-%, 2 wt-%, 3 wt-%, 4 wt-%, or 5 wt-% based on the total weight of the composition. For such embodiments, the total concentration of filler is at most 40 wt-%, preferably at most 20 wt-%, and more preferably at most 15 wt-% filler, based on the total weight of the composition.

Fillers may be selected from one or more of a wide variety of materials suitable for incorporation in compositions used for dental applications, such as fillers currently used in dental restorative compositions, and the like.

The filler can be an inorganic material. It can also be a crosslinked organic material that is insoluble in the polymerizable resin, and is optionally filled with inorganic filler. The filler is generally non-toxic and suitable for use in the mouth. The filler can be radiopaque, radiolucent, or nonradiopaque. Fillers as used in dental applications are typically ceramic in nature.

Suitable inorganic filler particles include quartz (i.e., silica), submicron silica, zirconia, submicron zirconia, and non-vitreous microparticles of the type described in U.S. 4,503,169 (Randklev).

The filler can also be an acid-reactive filler. Suitable acid-reactive fillers include metal oxides, glasses, and metal salts. Typical metal oxides include barium oxide, calcium oxide, magnesium oxide, and zinc oxide. Typical glasses include borate glasses, phosphate glasses, and fluoroaluminosilicate ("FAS") glasses. The FAS glass typically contains sufficient elutable cations so that a hardened dental composition will form when the glass is mixed with the components of the hardenable composition. The glass also typically contains sufficient elutable fluoride ions so that the hardened composition will have cariostatic properties. The glass can be made from a melt containing fluoride, alumina, and other glass-forming ingredients using techniques familiar to those skilled in the FAS glassmaking art. The FAS glass typically is in the form of particles that are sufficiently finely divided so that they can conveniently be mixed with the other cement components and will perform well when the resulting mixture is used in the mouth.

Generally, the average particle size (typically, diameter) for the FAS glass is no greater than 12 micrometers, typically no greater than 10 micrometers, and more typically no greater than 5 micrometers as measured using, for example, a sedimentation particle size analyzer. Suitable FAS glasses will be familiar to those skilled in the art, and are available from a wide variety of commercial sources, and many are found in currently available glass ionomer cements such as those commercially available under the trade designations VITREMER, VITREBOND, RELYX LUTING CEMENT, RELYX LUTING PLUS CEMENT, PHOTAC-FIL QUICK, KETAC-MOLAR, and KETAC-FIL PLUS (3M ESPE Dental Products, St. Paul, MN), FUJI II LC and FUJI IX (G-C Dental Industrial Corp., Tokyo, Japan) and CHEMFIL Superior (Dentsply International, York, PA). Mixtures of fillers can be used if desired.

Other suitable fillers are disclosed in US 6,387,981 (Zhang et al.) and 6,572,693 (Wu et al.) U.S. Patent No. 6,730,156 (Windisch et al.), US 6,899,948 (Zhang et al.), and US 7,393,882 (Wu et al.). Filler components described in these references include nanosized silica particles, nanosized metal oxide particles, and combinations thereof. Nanofillers are also described in U.S. Pat. Nos. 7,090,721 (Craig et al.), 7,090,722 (Budd et al.) and 7,156,911; and U.S. Patent No. 7649029 (Kolb et al.).

Examples of suitable organic filler particles include filled or unfilled pulverized polycarbonates, polyepoxides, poly(meth)acrylates and the like. Commonly employed dental filler particles are quartz, submicron silica, and non-vitreous microparticles of the type described in U.S. Pat. No. 4,503,169 (Randklev).

Mixtures of these fillers can also be used, as well as combination fillers made from organic and inorganic materials.

Fillers may be either particulate or fibrous in nature. Particulate fillers may generally be defined as having a length to width ratio, or aspect ratio, of 20:1 or less, and more commonly 10:1 or less. Fibers can be defined as having aspect ratios greater than 20: 1, or more commonly greater than 100:1. The shape of the particles can vary, ranging from spherical to ellipsoidal, or more planar such as flakes or discs. The macroscopic properties can be highly dependent on the shape of the filler particles, in particular the uniformity of the shape.

Micron-size particles are very effective for improving post-cure wear properties. In contrast, nanoscopic fillers are commonly used as viscosity and thixotropy modifiers. Due to their small size, high surface area, and associated hydrogen bonding, these materials are known to assemble into aggregated networks.

In some embodiments, the dental composition preferably comprises a nanoscopic particulate filler (i.e., a filler that comprises nanoparticles) having an average primary particle size of less than about 0.100 micrometers (i.e., microns), and more preferably less than 0.075 microns. As used herein, the term "primary particle size" refers to the size of a non-associated single particle. The average primary particle size can be determined by cutting a thin sample of hardened dental composition and measuring the particle diameter of about 50-100 particles using a transmission electron micrograph at a magnification of 300,000 and calculating the average. The filler can have a unimodal or polymodal (e.g., bimodal) particle size distribution. The nanoscopic particulate material typically has an average primary particle size of at least about 2 nanometers (nm), and preferably at least about 7 nm. Preferably, the nanoscopic particulate material has an average primary particle size of no greater than about 50 nm, and more preferably no greater than about 20 nm in size. The average surface area of such a filler is preferably at least about 20 square meters per gram (m²/g), more preferably, at least about 50 m²/g, and most preferably, at least about 100 m²/g.

In some preferred embodiments, the dental composition comprises silica nanoparticles. Suitable nano-sized silicas are commercially available from Nalco Chemical Co. (Naperville, IL) under the product designation NALCO COLLOIDAL SILICAS. For example, preferred silica particles can be obtained from using NALCO products 1040, 1041, 1042, 1050, 1060, 2327 and 2329.

Silica particles are preferably made from an aqueous colloidal dispersion of silica (i.e., a sol or aquasol). The colloidal silica is typically in the concentration of about 1 to 50 weight percent in the silica sol. Colloidal silica sols that can be used are available commercially having different colloid sizes, see Surface & Colloid Science, Vol. 6, ed. Matijevic, E., Wiley Interscience, 1973. Preferred silica sols for use making the fillers are supplied as a dispersion of amorphous silica in an aqueous medium (such as the Nalco colloidal silicas made by Nalco Chemical Company) and those which are low in sodium concentration and can be acidified by admixture with a suitable acid (e.g. Ludox colloidal silica made by E. I. Dupont de Nemours & Co. or Nalco 2326 from Nalco Chemical Co.).

Preferably, the silica particles in the sol have an average particle diameter of about 5-100 nm, more preferably 10-50 nm, and most preferably 12-40 nm. A particularly preferred silica sol is NALCO^{tm} 1042 or 2327.

In some embodiments, the dental composition comprises zirconia nanoparticles.

Suitable nano-sized zirconia nanoparticles can be prepared using hydrothermal technology as described in U.S. 7,241,437 (Davidson et al.).

In some embodiments, lower refractive index (e.g. silica) nanoparticles are employed in combination with high refractive index (e.g. zirconia) nanoparticles in order to index match (refractive index within 0.02) the filler to the refractive index of the polymerizable resin.

In some embodiments, the nanoparticles are in the form of nanoclusters, i.e. a group of two or more particles associated by relatively weak intermolecular forces that cause the particles to clump together, even when dispersed in a hardenable resin.

Preferred nanoclusters can comprise a substantially amorphous cluster of non-heavy (e.g. silica) particles, and amorphous heavy metal oxide (i.e. having an atomic number greater than 28) particles such as zirconia. The primary particles of the nanocluster preferably have an average diameter of less than about 100 nm. Suitable nanocluster fillers are described in U.S. 6,730,156 (Windisch et al.).

The curable dental compositions may include resin-modified glass ionomer cements such as those described in U.S. 5,130,347 (Mitra), U.S. 5,154,762 (Mitra), U.S. 5,925,715 (Mitra et al.) and 5,962,550 (Akahane). Such compositions can be powder-liquid, paste-liquid or paste-paste systems. Alternatively, copolymer formulations such as those described in US 6,126,922 (Rozzi) may also be included.

In some preferred embodiments the dental composition may further include an encapsulated particulate wherein the encapsulated particulate comprises a basic core material and an inorganic shell material comprising a metal oxide surrounding the core. In some embodiments, the basic core material is curable or hardenable, such as in the case of calcium silicate. In some embodiments, the composition further comprises at least one second filler, such as fluoroaluminosilicate (FAS) glass and/or a nanoscopic particulate filler. In some embodiments, the first and/or second part comprises a polymerizable material. Such particulates are described in Applicant's copending WO 2018/102484 (Christensen et al.).

An initiator is typically added to the mixture of polymerizable ingredients (i.e. curable resins and the addition-fragmentation agent of the invention). The initiator is sufficiently miscible with the resin system to permit ready dissolution in (and discourage separation from) the polymerizable composition. Typically, the initiator is present in the composition in effective amounts, such as from about 0.1 weight percent to about 5.0 weight percent, based on the total weight of the polymerizable components of the composition.

As previously described, the preferred initiators are Norrish Type II photoinitiators.

Curing of the dental compositions is affected by exposing the composition to a radiation source, preferably a visible light source. It is convenient to employ light sources that emit actinic radiation light between 250 nm and 800 nm (particularly blue light of a wavelength of 380-520 nm) such as quartz halogen lamps, tungsten-halogen lamps, mercury arcs, carbon arcs, low-, medium-, and high-pressure mercury lamps, plasma arcs, light emitting diodes, and lasers. In general, useful light sources have intensities in the range of 500-1500 mW/cm². A variety of conventional lights for hardening such compositions can be used.

The exposure may be accomplished in several ways. For example, the polymerizable composition may be continuously exposed to radiation throughout the entire hardening process (e.g., about 2 seconds to about 60 seconds). It is also possible to expose the composition to a single dose of radiation, and then remove the radiation source, thereby allowing polymerization to occur. In some cases materials can be subjected to light sources that ramp from low intensity to high intensity. Where dual exposures are employed, the intensity of each dosage may be the same or different. Similarly, the total energy of each exposure may be the same or different.

In some embodiments, the disclosure provides a universal restorative composite comprising:
a) 15-30 wt % of a curable dental resin comprising at least two polymerizable, ethylenically unsaturated groups;
b) 70-85 wt % of an inorganic filler, preferably a surface modified filler;
c) 0.1 to 10 parts by weight of the addition-fragmentation agent, relative to 100 parts by weight of a) and b), said curable composition further comprising an initiator and < 2%, stabilizers, pigments, etc.

In some embodiments, the disclosure provides a flowable restorative (flowable) composite comprising:
a) 25-50 wt % of a curable dental resin comprising at least two polymerizable, ethylenically unsaturated groups;
b) 50-75wt % of an inorganic filler, preferably a surface modified filler;
c) 0.1 to 10 parts by weight of the addition-fragmentation agent, relative to 100 parts by weight of a) and b), said curable composition further comprising an initiator and < 2% initiators, stabilizers, pigments, etc.

In some embodiments, the disclosure provides a resin modified glass-ionomer adhesive comprising:
a) 10-25 wt.% of a partially (meth)acrylated poly(meth) acrylic acid;
b) 5-20% of a hydroxyalkyl (meth)acrylate;
c) 30-60% of fluoroaluminosilicate (FAS) acid reactive glass
d) 0-20% non-acid reactive fillers, preferably surface-treated;
e) 10-20% water; and
f) 0.1 to 10 wt.% of the addition-fragmentation agent, relative to 100 parts by weight of a) and b) ),
g) said curable composition further comprising an initiator and < 2% stabilizers, pigments, etc.

Preferably the floroaluminosilicate is a silane methacrylate surface-treated floroaluminosilicate.

In some embodiments, the disclosure provides a dental adhesive comprising:
a) 30-8- wt.% mono (meth)acrylate) monomers;
b) 1-10 wt.% polyfunctional (meth)acrylate monomers;
c) 5-60 wt.%% monomers having a acid- functional group (including phosphate, phosphonate, carboxylate, sulfonic acids)
d) 0-10, preferably 1-10 wt.% poly(meth)acrylic acid methacrylate monomers;
e) 0.1 to 10 wt.% of the addition-fragmentation agent, relative to 100 parts by weight of a) to d);
f) an initiator,
g) 0-30% inorganic filler, preferably surface modified, relative to 100 parts by weight of a) to d);
h) 0 to 25 wt.% solvent relative to 100 parts by weight of a) to d);
i) 0 to 25 wt.% water relative to 100 parts by weight of a) to d); and
< 2% stabilizers, pigments, etc.

The composition may be polymerized with either a a photoinitiator system, a thermal initiator and/or a redox initiator system. Any conventional free radical initiator may be used to generate the initial radical. Typical thermal initiators include peroxides such as benzoyl peroxide and azo compounds such as azobisisobutyronitrile and dicumyl peroxide.

However, in some embodiments it is preferred that the initiator be a Norrish Type II photoinitiator (hydrogen abstraction). The photoinitiator group may be derived from a benzophenone, anthraquinone, 9-fluorenone, anthrone, xanthone, thioxanthone, acridone, dibenzosuberone, chromone, flavone, benzyl, and acetophenone compounds Such groups may be represented by:

| | | |
|---|---|---|
| 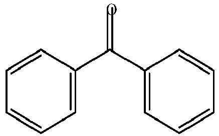 | 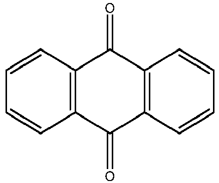 | 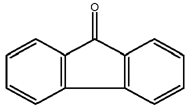 |
| 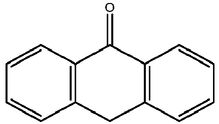 | 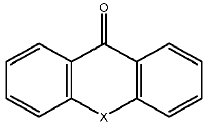 X = O, S or NH | 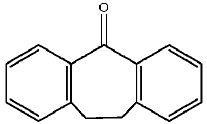 |
| 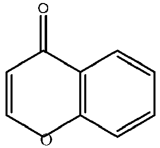 | 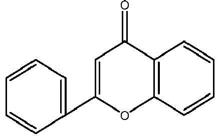 | 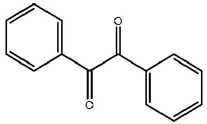 |
| 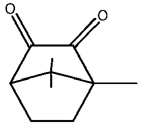 | | |

Preferably the photoinitiator is derived from an acetophenone, benzophenone, anthraquinone, 9-fluorenone, anthrone, xanthone, thioxanthone, acridone, dibenzosuberone, benzil, chromone, benzoyl cyclohexane, benzoyl piperidine, benzoyl piperazine, benzoyl morpholine, benzoyl tert-alkylene, camphorquinone and benzoyl-tert-hydroxyalkylene.

With Type II (abstraction type) photoinitiators, the requisite tertiary amine coinitiator group is pendent from the AFM agent to produce an initiating radical. The process of producing radicals is either through a hydrogen abstraction or an electron transfer mechanism depending on the coinitiator. The primary initiating radical is usually a radical centered on the coinitiator. In the presence of abstractable hydrogens (of the pendent amine coinitiators group) the reaction produces two radicals. The reaction pathway may be depicted with benzopheneone as follows:

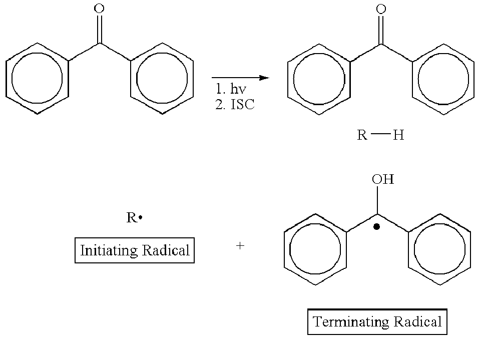

When the hydrogen donor source is an amine coinitiator, the excited state benzophenone participates in an electron transfer process forming the radical-anion/radical-cation pair. This is subsequently followed by a rapid proton-transfer from a carbon alpha to the nitrogen on the amine (aminyl radical) to the benzophenone radical-anion producing the semipinacol ketyl type radical and a carbon centered radical on the amine. The semipinacol ketyl type radical is not efficient at initiating polymerization, whereas the aminyl radical readily initiates polymerization. The products from the semipinacol ketyl type radical are still photoactive.

It will be understood that as the primary initiating radical is centred on the pendent tertiary amine groups, the AFM core will be integrated into the growing polymer to serve as an internal labile crosslink. 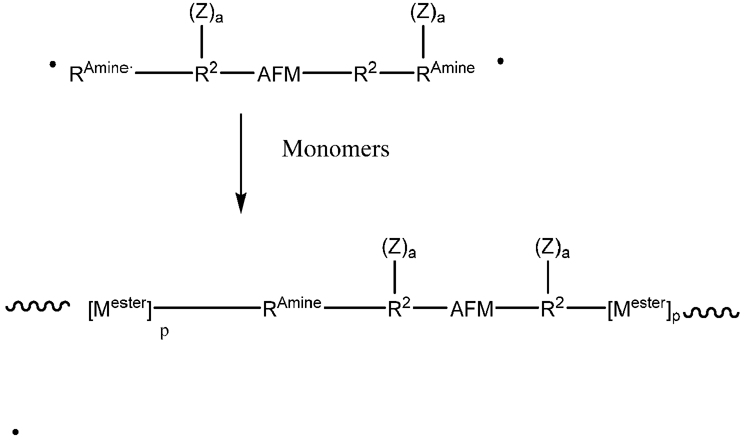 Where R^{Amine} is a aminyl radical (alpha to the nitrogen) which propagates polymerization with a monomer leading to the copolymer having an integral 1-methylene-3,3-dimethylpropylene AFM group. The M^{ester} monomer is shown, but any of the described monomers are useful. The ~ represent the remaining copolymer chain. The Z groups, if present, may serve as additional points polymerization and/or crosslinking.

In another embodiment the polymerizable composition comprises the addition-fragmentation agent, at least one polymerizable monomer, oligomer or resin, a peroxy catalyst, and a transition metal complex that participates in a redox cycle. The tertiary amine group pendent for the addition-fragmentation agent serves as a reducing agent in a redox polymerization cycle. The pendent tertiary amine reducing groups and oxidizing agents react with or otherwise cooperate with one another to produce free-radicals capable of initiating polymerization of the monomer or resin system (e.g., the ethylenically unsaturated component). This type of cure is a dark reaction, that is, it is not dependent on the presence of light and can proceed in the absence of light. The reducing and oxidizing agents are preferably sufficiently shelf-stable and free of undesirable colorization to permit their storage and use under typical conditions.

Redox reactions represent an important method for initiating the curing of acrylate, methacrylate and other vinyl-based resin, including adhesive and dental formulations. Redox-initiated curing often has advantages over photoinitiated curing, including improved depth of cure and a slower accumulation of stress during the initial stages of curing

Suitable oxidizing agents will also be familiar to those skilled in the art, and include but are not limited to persulfuric acid and salts thereof, such as sodium, potassium, ammonium, cesium, and alkyl ammonium salts. Additional oxidizing agents include peroxides such as benzoyl peroxides, hydroperoxides such as cumyl hydroperoxide, t-butyl hydroperoxide, and amyl hydroperoxide, as well as salts of transition metals such as cobalt (III) chloride and ferric chloride, cerium (IV) sulfate, perboric acid and salts thereof, permanganic acid and salts thereof, perphosphoric acid and salts thereof, and mixtures thereof.

It may be desirable to use more than one oxidizing agent or more than one reducing agent. Small quantities of transition metal compounds may also be added to accelerate the rate of redox cure. The reducing or oxidizing agents can be microencapsulated as described in U.S. 5,154,762 (Mitra et al.). This will generally enhance shelf stability of the polymerizable composition, and if necessary permit packaging the reducing and oxidizing agents together. For example, through appropriate selection of an encapsulant, the oxidizing and reducing agents can be combined with an acid-functional component and optional filler and kept in a storage-stable state.

Useful transition metal compounds have the general formula
[MLₚ]ⁿ⁺A⁻, wherein M is a transition metal that participates in a redox cycle,
L is a ligand, A- is an anion, n is the formal charge on the transition metal having a whole number value of 1 to 7, preferably 1 to 3, and p is the number of ligands on the transition metal having a number value of 1 to 9, preferably 1 to 2.

Useful transition metals, M, include the catalytically active valent states of Cu, Fe, Ru, Cr, Mo, Pd, Ni, Pt, Mn, Rh, Re, Co, V, Au, Nb and Ag. Preferred low valent metals include Cu(II), Fe(II), Ru(II) and Co(II). Other valent states of these same metals may be used, and the active low valent state generated *in situ.*

Useful anions, A⁻, include halogen, C₁ -C₆ alkoxy, NO₃²⁻, SO₄²⁻, PO₄³⁻, HPO₄²⁻, PF₆⁻, triflate, hexafluorophosphate, methanesulfonate, arylsulfonate, CN⁻ and alkyl carboxylates and aryl carboxylates.

The ligand, L, is used to solubilize the transition metal salts in a suitable solvent and adjust the redox potential of the transition metal for appropriate reactivity and selectivity. The ligands can direct the metal complex to undergo the desired one-electron atom transfer process, rather than a two-electron process such as oxidative addition/reductive elimination. The ligands may further enhance the stability of the complexes in the presence of different monomers and solvents or at different temperatures. Acidic monomers and monomers that strongly complex transition metals may still be efficiently polymerized by appropriate selection of ligands.

Useful ligands include those having one or more nitrogen, oxygen, phosphorus and/or sulfur atoms which can coordinate to the transition metal through a σ-bond, ligands containing two or more carbon atoms which can coordinate to the transition metal through a π-bond, and ligands which can coordinate to the transition metal through a µ-bond or an η-bond.

Useful ligands include those having one or more nitrogen, oxygen, phosphorus and/or sulfur atoms which can coordinate to the transition metal through a σ-bond are provided by monodentate and polydentate compounds preferably containing up to about 30 carbon atoms and up to 10 heteroatoms selected from aluminum, boron, nitrogen, sulfur, non-peroxidic oxygen, phosphorus, arsenic, selenium, antimony, and tellurium, where upon addition to the metal atom, following loss of zero, one, or two hydrogens, the polydentate compounds preferably forming with the metal, Mⁿ⁺, a 4-, 5-, or 6-membered saturated or unsaturated ring. Examples of suitable monodentate compounds or groups are carbon monoxide, alcohols such as ethanol, butanol, and phenol; pyridine, nitrosonium (i.e., NO⁺); compounds of Group Vb elements such as ammonia, phosphine, trimethylamine, trimethylphosphine, tributylphosphine, triphenylamine, triphenylphosphine, triphenylarsine, tributylphosphite; nitriles such as acetonitrile, benzonitrile; isonitriles such as phenylisonitrile, butylisonitrile; carbene groups such as ethoxymethylcarbene, dithiomethoxycarbene; alkylidenes such as methylidene and ethylidene.

Suitable polydentate compounds or groups include dipyridyl, 1,2-bis(diphenylphosphino)ethane, 1,2-bis(diphenylarsino)ethane, bis(diphenylphosphino)methane, polyamines such as ethylenediamine, propylenediamine, tetramethyl ethylene diamine, hexamethyl tris-aminoethylamine, diethylenetriamine, 1,3- diisocyanopropane, and hydridotripyrazolylborate; the hydroxycarboxylic acids such as glycolic acid, lactic acid, salicylic acid; polyhydric phenols such as catechol and 2,2'-dihydroxybiphenyl; hydroxyamines such as ethanolamine, propanolamine, and 2-aminophenol; dithiocarbamates such as diethyldithiocarbamate, dibenzyldithiocarbamate; xanthates such as ethyl xanthate, phenyl xanthate; the dithiolenes such as bis(perfluoromethyl)-1,2-dithiolene; aminocarboxylic acids such as alanine, glycine and o-aminobenzoic acid; dicarboxylic diamines as oxalamide, biuret; diketones such as 2,4-pentanedione; hydroxyketones such as 2-hydroxyacetophenone; alpha-hydroxyoximes such as salicylaldoxime; ketoximes such as benzil oxime; 1,10-phenanthroline, porphyrin, cryptands and crown ethers, such as 18-crown-6 and glyoximes such as dimethylglyoxime.

Other suitable ligands that can coordinate to the transition metal through a σ-bond are the inorganic groups such as, for example, F⁻, OH⁻, Cl⁻, Br⁻, I⁻, and H⁻ and the organic groups such as, for example, CN⁻, SCN⁻, acetoxy, formyloxy, benzoyloxy, and the like. The ligand can also be a unit of a polymer; for example the amino group in poly(ethyleneamine); the phosphino group in poly(4-vinylphenyldiphenylphosphine); the carboxylic acid group in poly(acrylic acid); and the isonitrile group in poly(4-vinylphenylisonitrile).

Useful ligands containing two or more carbon atoms which can coordinate to the transition metal through a π-bond are provided by any monomeric or polymeric compound having an accessible unsaturated group, i.e., an ethylenic, -C=C- group; acetylenic, -C≡C- group; or aromatic group which has accessible π-electrons regardless of the total molecular weight of the compound.

Illustrative of π-bond ligands are the linear and cyclic ethylenic and acetylenic compounds having less than 100 carbon atoms (when monomeric), preferably having less than 60 carbon atoms, and from zero to 10 heteroatoms selected from nitrogen, sulfur, non-peroxidic oxygen, phosphorous, arsenic, selenium, boron, aluminum, antimony, tellurium, silicon, germanium, and tin, the ligands being those such as ethylene, acetylene, propylene, methylacetylene, α-butene, 2-butene, diacetylene, butadiene, 1,2-dimethylacetylene, cyclobutene, pentene, cyclopentene, hexene, cyclohexene, 1,3-cyclohexadiene, cyclopentadiene, 1,4-cyclohexadiene, cycloheptene, 1-octene, 4-octene, 3,4-dimethyl-3-hexene, and 1-decene; η³-allyl, η³-pentenyl, norbornadiene, η⁵-cyclohexadienyl, cycloheptatriene, cyclooctatetraene, and substituted and unsubstituted carbocyclic and heterocyclic aromatic ligands having up to 25 rings and up to 100 carbon atoms and up to 10 hetero atoms selected from nitrogen, sulfur, non-peroxidic oxygen, phosphorus, arsenic, selenium, boron, aluminum, antimony, tellurium, silicon, germanium, and tin, such as, for example, η⁵-cyclopentadienyl, benzene, mesitylene, toluene, xylene, tetramethylbenzene, hexamethylbenzene, fluorene, naphthalene, anthracene, chrysene, pyrene, η⁷-cycloheptatrienyl, triphenylmethane, paracyclophane, 1,4-diphenylbutane, η⁵-pyrrole, η⁵-thiophene, η⁵-furan, pyridine, gamma-picoline, quinaldine, benzopyrane, thiochrome, benzoxazine, indole, acridine, carbazole, triphenylene, silabenzene, arsabenzene, stibabenzene, 2,4,6-triphenylphosphabenzene, η⁵-selenophene, dibenzostannepine, η⁵-tellurophene, phenothiazine, selenanthrene, phenoxaphosphine, phenarsazine, phenatellurazine, η⁵-methylcyclopentadienyl, η⁵-pentamethylcyclopentadienyl, and 1-phenylborabenzene. Other suitable aromatic compounds can be found by consulting any of many chemical handbooks.

Preferred ligands include unsubstituted and substituted pyridines and bipyridines, tertiary amines, including polydentate amines such as tetramethyl ethylenediamine and hexamethyl tris-aminoethylamine, acetonitrile, phosphites such as (CH₃O)₃P, 1,10-phenanthroline, porphyrin, cryptands and crown ethers, such as 18-crown-6. The most preferred ligands are polydentate amines, bipyridine and phosphites. Useful ligands and ligand-metal complexes useful in the initiator systems of the present invention are described in Matyjaszewski and Xia, Chem, Rev., vol. 101, pp. 2921-2990, 2001.

The molar proportion of tertiary amine reducing agent (of the AFM of the invention) relative to transition metal complex is generally that which is effective to polymerize the selected polymerizable components(s), but may be from 1000:1 to 5:1, preferably from 500:1 to 25:1, more preferably from 250:1 to 50:1, and most preferably from 200:1 to 75:1. The oxidant and photolabile reductant of the redox initiator system are used in approximately equimolar amount. Generally the mole ratio of the oxidant and photolabile reductant is from 1:1.5 to 1.5:1, preferably 1:1.1 to 1.1 to 1.

The reducing agent groups and oxidizing agents are present in amounts sufficient to permit an adequate free-radical reaction rate. This can be evaluated by combining all of the ingredients of the polymerizable composition except for the optional filler, and observing whether or not a hardened mass is obtained.

Preferably, the reducing agent groups are present in an amount of at least 0.01 part by weight, and more preferably at least 0.1 parts by weight, based on the total weight of the monomer components of the polymerizable composition. TH tertiary amine reducing agent groups may be calculated as a fraction of the addition-fragmentation agent. Preferably, the reducing agent is present in an amount of no greater than 10 parts by weight, and more preferably no greater than 5 parts by weight, based on the total weight of the polymerizable components of the polymerizable composition.

Preferably, the oxidizing agent is present in an amount of at least 0.01 part by weight, and more preferably at least 0.10 part by weight, based on the total weight of the polymerizable components of the polymerizable composition. Preferably, the oxidizing agent is present in an amount of no greater than 10 part by weight, and more preferably no greater than 5 parts by weight, based on the total weight of the polymerizable components of the polymerizable composition.

The curable composition may also include other additives. Examples of suitable additives include tackifiers (e.g., rosin esters, terpenes, phenols, and aliphatic, aromatic, or mixtures of aliphatic and aromatic synthetic hydrocarbon resins), surfactants, plasticizers (other than physical blowing agents), nucleating agents (e.g., talc, silica, or TiO₂), pigments, dyes, reinforcing agents, solid fillers, stabilizers (e.g., UV stabilizers), and combinations thereof. The additives may be added in amounts sufficient to obtain the desired properties for the cured composition being produced. The desired properties are largely dictated by the intended application of the resultant polymeric article article.

Adjuvants may optionally be added to the compositions such as colorants, abrasive granules, antioxidant stabilizers, thermal degradation stabilizers, light stabilizers, conductive particles, tackifiers, flow agents, bodying agents, flatting agents, inert fillers, binders, blowing agents, fungicides, bactericides, surfactants, plasticizers, rubber tougheners and other additives known to those skilled in the art. They also can be substantially unreactive, such as fillers, both inorganic and organic. These adjuvants, if present, are added in an amount effective for their intended purpose.

In some embodiments, a toughening agent may be used. The toughening agents which are useful in the present invention are polymeric compounds having both a rubbery phase and a thermoplastic phase such as: graft polymers having a polymerized, diene, rubbery core and a polyacrylate, polymethacrylate shell; graft polymers having a rubbery, polyacrylate core with a polyacrylate or polymethacrylate shell; and elastomeric particles polymerized in situ in the epoxide from free radical polymerizable monomers and a copolymerizable polymeric stabilizer.

In some embodiments the crosslinkable composition may include filler. In some embodiments the total amount of filler is at most 50 wt.%, preferably at most 30 wt.%, and more preferably at most 10 wt.% filler. Fillers may be selected from one or more of a wide variety of materials, as known in the art, and include organic and inorganic filler. Inorganic filler particles include silica, submicron silica, zirconia, submicron zirconia, and non-vitreous microparticles of the type described in U.S. Pat. No. 4,503,169 (Randklev).

Filler components include nanosized silica particles, nanosized metal oxide particles, and combinations thereof. Nanofillers are also described in U.S. 7,090,721 (Craig et al.), 7,090,722 (Budd et al.), 7,156,91 1(Kangas et al.), and 7,649,029 (Kolb et al.).

In some embodiments the filler may be surface modified. A variety of conventional methods are available for modifying the surface of nanoparticles including, e.g., adding a surface-modifying agent to nanoparticles (e.g., in the form of a powder or a colloidal dispersion) and allowing the surface-modifying agent to react with the nanoparticles. Other useful surface-modification processes are described in, e.g., U.S. Pat. No. 2,801,185 (Iler), U.S. Pat. No. 4,522,958 (Das et al.) U.S. 6,586,483 (Kolb et al.).

The present addition fragmentation agents are also useful in the preparation of hardcoats. The term "hardcoat" or "hardcoat layer" means a layer or coating that is located on the external surface of an object, where the layer or coating has been designed to at least protect the object from abrasion. The present disclosure provides hardcoat compositions comprising the addition-fragmentation agent of the invention and, a multi-functional (moth)acrylate monomer comprising three or more (meth)acrylate groups, and/or a multi- functional (meth)acrylate oligomer and optionally a (meth)acrylate-functional diluent.

Useful multifunctional (meth)acrylate monomers comprise three or more (meth)acrylate groups. Multifunctional (meth)acrylate monomers are useful in the practice of the present invention because they add abrasion resistance to the hard coat layer. Preferred multifunctional (meth)acrylate monomers comprising three or more (meth)acrylate groups include trimethylol propane tri(meth)acrylate (TMPTA), pentaerythritol tri(meth)acrylate, pentaerythritol tetra(meth)acrylate, dipentrithritol tri(meth)acrylate (Sartomer 355), dipentaerythritol penta(meth)acrylate (Sartomer 399), dipentaerythritol hydroxy penta(meth)acrylate (DPHPA), glyceryl propoxy tri(meth)acrylate, trimethylopropane tri(meth)acrylate, and mixtures thereof Another useful radiation-curable component of the present invention is the class of multifunctional (meth)acrylate oligomers, having two or more (meth)acrylate groups, and having an average molecular weight (Mw) in the range from about 400 to 2000.

Useful multi-functional (meth)acrylate oligomers include polyester (meth)acrylates, polyurethane (meth)acrylates, and (meth)acrylated epoxy (meth)acrylates. (Meth)acrylated epoxy (meth)acrylates and polyester(meth)acrylates are most preferred because they tend to have a relatively low viscosity and therefore allow a more uniform layer to be applied by the spin coating method. Specifically, preferred multifunctional (meth)acrylate oligomers include those commercially available from UCB Radcure, Inc. of Smyrna, Georgia and sold under the trade name Ebecryl (Eb): Eb40 (tetrafunctional acrylated polyester oligomer), ENO (polyester tetra-functional (meth)acrylate oligomer), Eb81 (multifunctional (meth)acrylated polyester oligomer), Eb600 (bisphenol A epoxy di(meth)acrylate), Eb605 (bisphenol A epoxy di(meth)acrylate diluted with 25% tripropylene glycol di(meth)acrylate), Eb639 (novolac polyester oligomer), Eb2047 (trifunctional acrylated polyester oligomer), Eb3500 (di- functional Bisphenol-A oligomer acrylate), Eb3604 (multi-functional polyester oligomer acrylate), Eb6602 (trifunctional aromatic urethane acrylate oligomer), Eb8301 (hexafunctional aliphatic urethane acrylate), EbW2 (difunctional aliphatic urethane acrylate oligomer), and mixtures thereof. Of these, the most preferred are, Eb 600, Eb605, Eb80, and Eb81.

The (meth)acrylate-functional diluents, also referred to herein as "reactive diluents", are relatively low molecular weight mono- or di-functional, non-aromatic, (meth)acrylate monomers. These relatively low molecular weight reactive diluents are advantageously of a relatively low viscosity, e.g., less than about 30 centipoise (cps) at 25°C. Di-functional, non-aromatic (meth)acrylates are generally preferred over mono- functional non-aromatic (meth)acrylates because di-functional non-aromatic (meth)acrylates allow for quicker cure time. Preferred reactive diluents include 1,6-hexanediol di(meth)acrylate (HDDA from UCB Radcure, Inc. of Smyrna, Georgia), tripropylene glycol di(meth)acrylate, isobornyl (meth)acrylate (1130A, Radcure), 2(2-ethoxyethoxy) ethyl (meth)acrylate (sold under the trade name Sartomer 256 from SARTOMER Company, Inc. of Exton, Pennsylvania), n-vinyl formamide (Sartomer 497), tetrahydrofurfuryl (meth)acrylate(Sartomer 285), polyethylene glycol di(meth)acrylate (Sartomer 344), tripropylene glycol di(meth)acrylate (Radcure), neopentyl glycol dialkoxy di(meth)acrylate, polyethyleneglycol di(meth)acrylate, and mixtures thereof.

The hardcoat composition may comprise:
0.1 - 10 wt.% of the addition fragmentation agent of the invention;
20-80 wt.% of multifunctional (meth)acrylate monomers and/or multifunctional (meth)acrylate oligomers,
0 to 25 wt.% range of (meth)acrylate diluent, (0-25 wt.%)
20 to 75 wt.% of silica. The weight ranges referring to the silica *per se,* whether or not functionalized.

### Examples

Unless otherwise noted, all parts, percentages, ratios, etc. in the Examples and the rest of the specification are by weight. Unless otherwise indicated, all other reagents were obtained, or are available from fine chemical vendors such as Sigma-Aldrich Company, St. Louis, Missouri, or may be synthesized by known methods. Table 1 (below) lists materials used in the examples and their sources.

**TABLE 1. Materials List**

| DESIGNATION | DESCRIPTION | SOURCE |
|---|---|---|
| EDMAB | Ethyl 4-(dimethylamino)benzoate | Sigma-Aldrich, St. Louis, MO |
| Ethylene glycol | Ethylene glycol | Sigma-Aldrich, St. Louis, MO |
| H2SO4 | Sulfuric acid | J.T.Baker Avantor Performance Materials Center Valley, PA USA |
| NaOH | Sodium hydroxide | Sigma-Aldrich |
| Ethyl acetate | Ethyl acetate | Sigma-Aldrich |
| Heptane | Heptane | Sigma-Aldrich |
| AFM1 | AFM monomer prepared as described in US 9056043 (Joly et al.) | 3M Oral Care Solutions Division - Seefeld - Germany |
| Succinic anhydride | Succinic anhydride | Sigma-Aldrich |
| DMAP | 4-dimethylamine pyridine | Alfa Aesar, Haverhill, MA |
| BHT | Butylated hydroxy toluene | Sigma-Aldrich |
| | | |
| DMAPE | 2-[4-(dimethylamino)phenyl]ethanol | 3M Company |
| DCC | dicyclohexyl carbodiimide | Alfa Aesar, Haverhill, MA |
| NaHCO₃ | Sodium bicarbonate | Sigma-Aldrich |
| Na₃SO₄ | Sodium sulfate | Sigma-Aldrich |
| AFM precursor | 2,2-dimethyl-4-methylene-pentanedioic acid | 3M Oral Care Solutions Division - Seefeld - Germany |
| S/T SilicalZirconia Clusters | SAT SilicalZirconia Clusters refers to silane-treated silica-zirconia nanocluster filler, prepared generally as described in U.S. Pat. No. 6730156 at column 25, lines 50-63 (Preparatory Example A) and at column 25, line 64 through column 26, line 40 (Preparatory Example B) with minor modifications, including performing the silanization in 1-methoxy-2-propanol (rather than water) adjusted to a pH of ~8.8 with aqueous NH₄OH (rather than to a pH of 3-3.3 with trifluoroacetic acid), and obtaining the nanocluster filler by gap drying (rather than spray drying). | 3M Oral Care Solutions Division, Irvine, CA, USA |
| Crystal s/t nanozirconia filler | Crystal s/t nanozirconia filler refers to Nanozirconia filler-silane-treated nanozirconia powder was prepared as described in U.S. Pat. No. 7,156,911, Preparatory Example 1A except that GF-3 1 silane was used instead of SILQUEST A-1230 | 3M Oral Care Solutions Division, Irvine, CA, USA |
| 20 nm Supreme s/t/ silica filler | 20 nm Supreme s/t/ silica filler refers to 20 nm silica particles that was surface treated with methacrylates | 3M Oral Care Solutions Division, Irvine, CA, USA |
| Sukgyung 100 nm | Sukgyung 100 nm YbF3 refers to Ytterbium | Sukgyung AT |
| YbF3 | fluoride | Co. Ltd., (Korea); |
| ERGP-IEM | Prepared as described in the Example section of EP Patent Publication Number EP 2401998 | 3M Oral Care Solutions Division - Seefeld - Germany |
| UDMA | Urethane dimethacrylate obtained under the trade designation ROHAMERE 6661-0 (CAS No. 41137-60-4) | Rohm Tech, Inc., Malden, MA, USA |
| DDDMA | Dodecanediol dimethacrylate | Sartomer Co., Inc. Exton, PA |
| | | |
| | | |
| CPQ | camphorquinone | Sigma Aldrich |
| BZT | 2-(2'-hydroxy-5'-methacryloxyethylphenyl)-2H-benzotriazole | Sigma-Aldrich |
| Iodonium | Diphenyliodonium hexafluorophosphate | Alfa Aesar, Haverhill, MA |
| GDMA | Glycerol dimethacrylate | Sigma-Aldrich |
| HEMA | Hydroxyethyl methacrylate | Sigma-Aldrich |
| Potassium persulfate | Potassium persulfate | Sigma-Aldrich |
| Coarse FAS filler | Coarse FAS glass with silane treatment having a particle size of about 3 micrometers and treated as described in U.S. Pat. No. 7173074, column 13 | Mo-Sci Corp., Columbia, MO |
| Fine FAS filler | Fine FAS glass with silane treatment having a particle size of about I micrometer and treated as described in U.S. Pat. No. 7173074, column 13 | Mo-Sci Corp. |
| R812S fumed silica | Hydrophobic fumed silica obtained under the trade designation AEROSIL R 812 S | Degussa, Parsippany, NJ |
| Portland cement | White Portland cement | Federal White Cement, Ontario, Canada |
| Al₂O₃ Coated Portland cement | In 80945US002 page 36, example 2 | 3M Co., Maplewood, MN |
| 3378 Si/Zr filler | silane treated zirconia-silica filler prepared as described in U.S. Pat. No. 6818682 at col. 11, line 41 through col. 12, line 10. | 3M Co. |
| AF-DMAPE | Prepared in this invention | 3M Co. |
| AFM-1 Di-DMAPE | Prepared in this invention | 3M Co. |
| VBCP | Reaction product of 2-isocyanatoethyl methacrylate and a copolymer of acrylic acid and itaconic acid prepared as described in U.S. Pat. No. 6130347, Example 11. | 3M Co. |
| BHT | Butylated hydroxy toluene | Sigma-Aldrich |
| CE-4 | Comparative cement product (CE-4) for mechanical testing obtained under the trade designation RELYX LUTING PLUS CEMENT PRODUCT from 3M Co., Maplewood, MN. | 3M Co. |
| HEDMAB | 2-hydroxyethyl 4-(dimethylamino)benzoate prepared as described below. | |
| AFM Succinate | AFM succinate prepared as described below. | |
| AFM-1 Di-DMAPE | prepared as described below. | |
| AF-DMAPE | prepared as described below. | |
| AFM-HEDMAB Adduct | prepared as described below. | |

### Test Methods and Material Preparation

### NUCLEAR MAGNETIC RESONANCE (NMR) TEST METHOD

Approximately 50 milligrams sample were dissolved in appropriate deuterated solvents. Proton (¹H) and carbon (¹³C) NMR spectra were acquired on a Bruker AVANCE III 500 MHz spectrometer (Billerica, MA, USA) equipped with a broadband cryoprobe.

### CUSP DEFLECTION STRESS TEST METHOD

To measure stress development during the curing process, a slot was machined into a rectangular 1 5x8x8 mm aluminum block, as shown in FIG. 1. The slot was 8 mm long, 2.5 mm deep, and 2 mm across, and was located 2 mm from an edge, 55 thus forming a 2mm wide aluminum cusp adjacent to a 2mm wide cavity containing dental compositions being tested. A linear variable displacement transducer (Model GT 1000, used with an E309 analog amplifier, both from RDP Electronics, United Kingdom) was positioned as shown to measure 60 the displacement of the cusp tip as the dental composition photocured at room temperature. Prior to testing, the slot in the aluminum block was sandblasted using Rocatec Plus Special Surface Coating Blasting Material (3M ESPE), treated with RelyX Ceramic Primer (3M ESPE), and finally treated 65 with a dental adhesive, Adper Easy Bond (3M ESPE). The slot was fully packed with the mixtures shown in the tables, which equalled approximately 100 mg of material. The material was irradiated for 1 minute with a dental curing lamp (Elipar S-b, 3M ESPE) positioned almost in contact, (<1 mm) with the material in the slot, then the displacement of the cusp in microns was recorded 9 minutes after the lamp was extinguished.

### DEPTH OF CURE TEST METHOD

The depth of cure was determined by filling a 10 millimeter stainless steel mold cavity with the composite, covering the top and bottom of the mold with sheets of polyester film, pressing the sheets to provide a leveled composition surface, placing the filled mold on a white background surface, irradiating the dental composition for 20 seconds using a dental curing light (3M Dental Products Curing Light 2500or 3M ESPE Elipar FreeLight2, 3M ESPE Dental Products), separating the polyester films from each side of the mold, gently removing (by scraping) materials from the bottom of the sample (i.e., the side that was not irradiated with the dental curing light), and measuring the thickness of the remaining material in the mold. The reported depths are the actual cured thickness in millimeters divided by 2.

### DIAMETRAL TENSILE STRENGTH TEST METHOD FOR PHOTOINITIATED EXAMPLES (CONTROLS 2 and 3 (CT-2 and CT-3) and EXAMPLES 2 to 8 (EX-2 to EX-8))

For Controls 2 and 3 (CT-2 and CT-3) and Examples 2 to 8 (EX-2 to EX-8), diametral tensile strength of a test sample was measured according to the following procedure. An uncured composite sample was injected into a 4-mm (inside diameter) glass tube; the tube was capped with silicone rubber plugs. The tube was compressed axially at approximately 2.88 kg/cm2 pressure for 5 minutes. The sample was then light cured for 80 seconds by exposure to a XL 1500 dental curing light (3M Company, St. Paul, Minn.), followed by irradiation for 90 seconds in a Kulzer UniXS curing box (Heraeus Kulzer GmbH, Germany). Cured samples were allowed to stand for 1 hour at about 37° C./90%+Relative Humidity. The sample was cut with a diamond saw to form disks about 2.2mm thick, which were stored in distilled water at 37° C. for about 24 hours prior to testing. Measurements were carried out on an Instron tester (Instron 4505, Instron Corp., Canton, Mass.) with a 10 kilonewton (kN) load cell at a crosshead speed of 1 mm/minute according to ISO Specification 7489 (or American Dental Association (ADA) Specification No. 27). Six disks of cured samples were prepared and measured with results reported in MPa as the average of the six measurements.

### DIAMETRAL TENSILE STRENGTH AND COMPRESSIVE STRENGTH TEST METHOD FOR REDOX EXAMPLES (EXAMPLE 30 (EX-30))

For Example 30 (EX-30), diametral tensile strength (DTS) and compressive strength (CS) of a test sample was measure according to the following procedure. Samples were prepared by mixing paste A and B with ratio of 1.5:1 on a paper mixing pad with a spatula for 20 seconds. Well mixed pastes were then transferred into a syringe and subsequently extruded into 4 millimeter (mm) glass tubes and cured at room temperature at 0.27 MPa (40 psi) pressure for 20 minutes, followed by further curing for 1 hour at 37° C and 97% relative humidity, and finally stabilizing at 37 °C in DI water for 24 hours. The sample was then cut using a diamond saw and tested on an INSTRON universal tester (Instron Corp., Canton, MA). For DTS measurements, seven such cured samples were cut to a length of 2 mm. DTS was determined according to ISO Standard 7489 using an INSTRON universal tester operated at a crosshead speed of 1 millimeter per minute (mm/min). For CS measurements, five such cured samples were cut to a length of 7 mm. CS was determined according to ISO Standard 7489 using an INSTRON universal tester operated at a crosshead speed of 1 millimeter per minute (mm/min).

### PREPARATION OF 2-HYDROXYETHYL 4-(DIMETHYLAMINO)BENZOATE (HEDMAB)

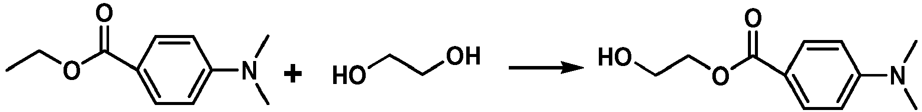

Ethyl 4-(dimethylamino)benzoate (EDMAB) (5 grams (g)) was suspended in ethylene glycol (50 g) in a 250 milliliter (mL) 3-neck round bottom flask equipped with a magnetic stirring bar under atmospheric nitrogen. With continuous stirring, sulfuric acid catalyst (1.5 g) was added. The mixture was heated at 100 °C for 24 hours. After 24 hours, the heat was turned off and cooled to room temperature. The reaction mixture was then transferred into a beaker containing excess 10% NaOH solution (100 mL). The cloudy solution was extracted with ethyl acetate (3 times, using 100 mL each wash). The ethyl acetate solution was washed several times with water (5 times, using 100 mL each wash). The organic layer was concentrated in a rotary evaporator. The obtained residue was crystallized from heptane to give a 2.3 g (42%) white solid. ¹H NMR was recorded and found to be consistent with the desired product.

### PREPARATION OF AFM SUCCINATE

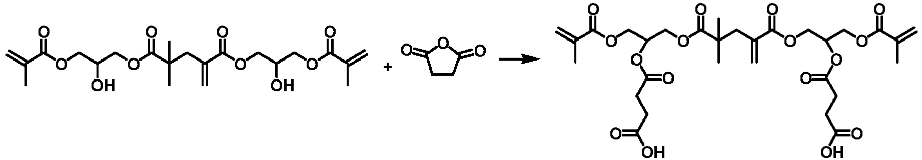

AFM-1 (5.95 g, 0.013 mol), succinic anhydride (2.55 g, 0.255 mol) DMAP (80 mg) BHT (8 mg) were charged into a 15 50 mL round-bottom flask equipped with a magnetic stirring bar and a dry air blanket. The flask was heated in an oil bath at 95-100° C. with continuous stirring for 5 hours. The heat was turned off and the product was collected with essentially 100% yield as a clear light yellow liquid. The structure of 20 AFM-succinate was confirmed by ¹H and ¹³C NMR.

### PREPARATION OF AFM-1 Di-DMAPE (ESTERIFICATION GENERAL PRODECURE)

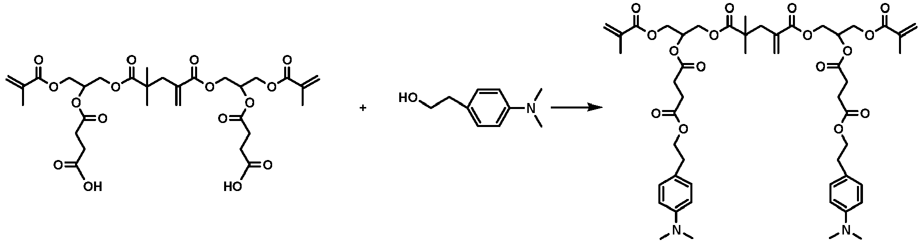

AFM succinate (39.92 g, 60.80 mmol) and DMAPE (20.01 g, 121.1 mmol) were suspended in ethyl acetate (300 mL) in 1 liter (L) 3-neck flask equipped with a mechanical stirrer, a thermocouple, and a dropping funnel with a nitrogen gas (N₂) stream applied via the dropping funnel then to a bubbler. 4-Dimethylamine pyridine (DMAP 1.5 g, 12 mmol) was added. With continuous stirring, the flask was cooled in an ice bath to 0-5 °C. A solution of dicyclohexyl carbodiimide (DCC, 25.5 g, 121 mmol) in ethyl acetate (100 mL) was added to the cold solution via the dropping funnel over 45 minutes. The reaction mixture was stirred at 0 °C for 2 hours then at room temperature overnight. Any solid formed was removed by filtration. The filtrate was extracted with 1 normal (N) HCl (1 time, with 100 mL), 10% aqueous NaHCO₃ (1 time, with100 mL) and water (1 time, with 100 mL). The organic layer was dried (Na₂SO₄) then concentrated in a rotary evaporator. After further drying, the product was obtained as yellow oil (51.4 g = 88.9% yield). Product structure was confirmed by ¹H NMR.

### PREPARATION OF AF-DMAPE

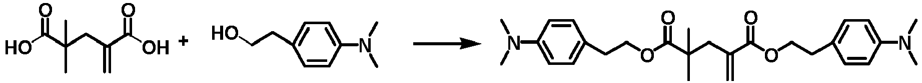

AF-DMAPE was prepared using the esterification general procedure (above) from AF (AFM precursor, 10 g, 58.08 mmol) and DMAPE (19.2 g, 116 mmol). The product was isolated as a yellow liquid (24.1 g = 88.9% yield). Product structure was confirmed by ¹H NMR.

### PREPARATION OF AFM-HEDMAB ADDUCT

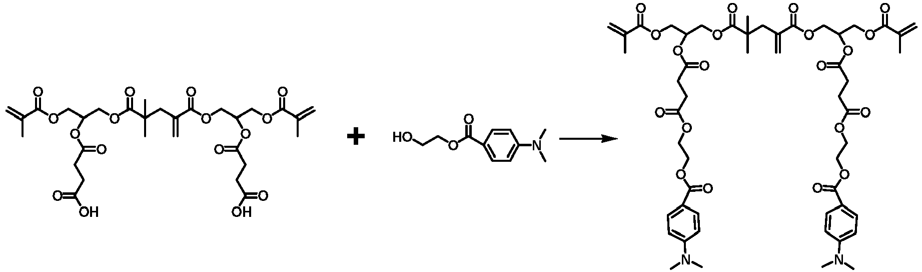

AFM-HEDMAB adduct was prepared following the general procedure from AFM succinate (22 g, 33.51 mmol) and HEDMAB (14 g, 66.91 mmol). The product was isolated as a yellow oil (29.6 g = 85% yield) and confirmed by ¹H NMR.

### Examples

### PHOTOINITIATED EXAMPLES

Resin formulation were compounded in two stages. In the first stage, a Base Resin 1 was prepared according to Table 2. EDMAB, AFM1 and the AFM-T-amine hybrid compounds above (i.e., AFM-1 Di-DMAPE, AF-DMAPE, and AFM-HEDMAB Adduct) were then added to the Base Resin A according to Tables 3, 4, and 5 to prepare the activated resin and paste formulations.

**TABLE 2. Base Resin 1 Formulation**

| MATERIAL | Base Resin 1 |
|---|---|
| ERGP-IEM, wt% | 68.83 |
| UDMA, wt% | 18.77 |
| DDDMA, wt% | 8.66 |
| CPQ, wt% | 0.28 |
| BZT, wt% | 0.5 |
| BHT, wt% | 0.05 |
| Iodonium, wt% | 0.3 |

**TABLE 3. Activated Resin Formulations**

| MATERIAL | CT-1A | EX-1A | EX-1B | EX-1C | EX-1D | EX-1F | EX-1G | EX-1H |
|---|---|---|---|---|---|---|---|---|
| Base Resin 1, wt% | 97.4 | 98.4 | 96.6 | 96.88 | 95 | 90 | 95 | 90 |
| AFM 1 Monomer, wt% | 1.5 | | | | | | | |
| EDMAB, wt% | 1.1 | | | | | | | |
| AFM-1 Di-DMAPE, wt% | | 1.6 | | | 5 | 10 | | |
| AFM 1-HEDMAB Adduct, wt% | | | 3.4 | | | | 5 | 10 |
| AF-DMAPE, wt% | | | | 3.12 | | | | |

**TABLE 4. Photoinitiated Paste Formulations**

| MATERIAL | CT-2 | EX-2 | EX-3 | EX-4 | EX-5 |
|---|---|---|---|---|---|
| Base Resin 1, wt% | CT-1A, 23.6 | EX-1A, 23.6 | EX-1B, 23.6 | EX-1C, 23.6 | EX-1D, 23.6 |
| S/T SilicalZirconia Clusters, wt% | 65.7 | 65.7 | 65.7 | 65.7 | 65.7 |
| Crystal s/t nanozirconia filler, wt% | 4.7 | 4.6 | 4.6 | 4.7 | 4.6 |
| 20 nm Supreme s/t/ silica filler, wt% | 1.9 | 1.9 | 1.9 | 1.8 | 1.9 |
| Sukgyung 100 nm YbF3, wt% | 4.2 | 4.2 | 4.2 | 4.2 | 4.2 |

**TABLE 5. Photoinitiated Paste Formulations**

| MATERIAL | CT-3 | EX-6 | EX-7 | EX-8 |
|---|---|---|---|---|
| Base Resin 1, wt% | CT-1A, 23.6 | EX-1F, 23.6 | EX-1G, 23.6 | EX-1H, 23.6 |
| S/T SilicalZirconia Clusters, wt% | 65.7 | 65.7 | 65.7 | 65.7 |
| Crystal s/t nanozirconia filler, wt% | 4.7 | 4.6 | 4.7 | 4,65 |
| 20 nm Supreme s/t/ silica filler, wt% | 1.9 | 1.9 | 1.8 | 1.85 |
| Sukgyung 100 nm YbF3, wt% | 4.2 | 4.2 | 4.2 | 4.2 |

**TABLE 6. Mechanical Properties of Photoinitiated Examples**

| EXAMPLE | Cusp Deflection, microns | Depth of Cure, millimeters | Diametral Tensile Strength, MPa |
|---|---|---|---|
| CT-2 | 8.70 +/- 0.48 | 5.23 +/- 0.15 | 77.65 +/- 2.95 |
| CT-3 | 8.52 +/- 0.21 | 5.52 +/- 0.10 | 81.14 +/- 1.52 |
| EX-2 | 6.75 +/- 0.11 | 4.92 +/- 0.28 | 75.92 +/- 4.35 |
| EX-3 | 7.58 +/- 0.28 | 5.00 +/- 0.25 | 77.73 +/- 2.12 |
| EX-4 | 8.80 +/- 0.22 | 5.04 +/- 0.42 | 78.10 +/- 3.61 |
| EX-5 | 7.24 +/- 0.38 | 5.54 +/- 0.13 | 79.82 +/- 2.12 |
| EX-6 | 6.47 +/- 0.38 | 5.37 +/- 0.19 | 75.79 +/- 5.67 |
| EX-7 | 7.17 +/- 0.10 | 5.49 +/-0.13 | 76.05 +/- 5.64 |
| EX-8 | 6.20 +/- 0.14 | 5.55 +/- 0.21 | 75.17 +/- 9.06 |

### REDOX EXAMPLES

### Redox Resin A Preparation:

All chemicals were added according to Table 7 into a large speed mixing cup (100 gram cup) and mixed at 3000 RPM (revolutions per minute) for 2 cycles lasting 2 minutes each. After mixing, the resin was ready for preparation of Paste A.

**TABLE 7. Redox Resin A Formulation**

| MATERIAL | REDOX RESIN A-1 | REDOX RESIN A-2 |
|---|---|---|
| GDMA, wt% | 28.4 | 19.0 |
| UDMA, wt% | 0.0 | 9.5 |
| HEMA, wt% | 71.1 | 71.1 |
| BHT, wt% | 0.1 | 0.1 |
| CPQ, wt% | 0.4 | 0.4 |

### Redox Resin B Preparation:

All chemicals were added according to Table 8 into a large speed mixing cup (100 gram cup) and mixed well to dissolve the VBCP in the resin. The materials were mixed at 3000 RPM for several cycles lasting 2 minutes each. After mixing, the resin was ready for preparation of Paste B.

**TABLE 8. Redox Resin B Formulation**

| MATERIAL | REDOX RESIN B-1 | REDOX RESIN B-2 |
|---|---|---|
| DI water, wt% | 38.7 | 38.0 |
| HEMA, wt% | 21.0 | 21.0 |
| VBCP, wt% | 40.3 | 41.0 |
| BHT, wt% | 0.1 | 0.0 |
| MEHQ, wt% | 0.0 | 0.1 |

### Redox Paste A Preparation:

Paste A mixtures were prepared by mixing Redox Resin A with additional components (as listed in Table 9) in a speed mixing cup at 25 gram level. The mixture was speed mixed at 3000 RPM for 2 cycles lasting 2 minutes each to form a uniform paste.

**TABLE 9. Redox Curing Compositions for Paste A**

| MATERIAL | EX-A1 | EX-A2 | EX-A3 | EX-A4 | EX-A5 | EX-A6 | EX-A7 | EX-A8 | EX-A9 | EX-A10 | EX-A11 |
|---|---|---|---|---|---|---|---|---|---|---|---|
| Redox Resin A-1, wt% | 0.0 | 0.0 | 0.0 | 0.0 | 28.45 | 28.45 | 28.45 | 28.45 | 28.45 | 28.45 | 28.45 |
| Redox Resin A-2, wt% | 28.83 | 28.83 | 28.83 | 28.83 | 0.0 | 0.0 | 0.0 | 0.0 | 0.0 | 0.0 | 0.0 |
| Potassium persulfate, wt% | 1.5 | 1.5 | 1.5 | 1.5 | 1.5 | 1.5 | 1.5 | 1.5 | 1.5 | 1.5 | 1.5 |
| coarse FAS filler, wt% | 30.0 | 38.0 | 60.0 | 55.0 | 30.0 | 38.0 | 60.0 | 38.0 | 60.0 | 55.0 | 33.0 |
| fine FAS filler, wt% | 38.0 | 30.0 | 0.0 | 0.0 | 38.0 | 30.0 | 0.0 | 0.0 | 0.0 | 0.0 | 0.0 |
| R812S fume silica, wt% | 2.0 | 2.0 | 2.0 | 2.0 | 2.0 | 2.0 | 2.0 | 2.0 | 2.0 | 2.0 | 2.0 |
| Portland cement, wt% | 0.0 | 0.0 | 8.0 | 13.0 | 0.0 | 0.0 | 8.0 | 30.0 | 0.0 | 0.0 | 0.0 |
| Al₂O₃ Coated Portland cement, wt% | 0.0 | 0.0 | 0.0 | 0.0 | 0.0 | 0.0 | 0.0 | 0.0 | 8.0 | 13.0 | 35.0 |

### Redox Paste B Preparation:

Paste B mixtures were prepared by mixing Redox Resin B with additional components (as listed in Table 10) in a speed mixing cup at 25 gram level. The mixture was speed mixed at 3000 RPM for 2 cycles lasting 2 minutes each to form a uniform paste.

**TABLE 10. Redox Curing Compositions for Paste B**

| MATERIAL | EX - B 1 | EX-B2 | EX-B3 |
|---|---|---|---|
| Redox Resin B-1 | 62.33 | 57.41 | 0.0 |
| Redox Resin B-2 | 0.0 | 0.0 | 55.33 |
| AF-DMAPE | 0.5 | 0.0 | 1.0 |
| AFM-1 Di-DMAPE | 0.0 | 2.0 | 1.0 |
| 3378 Si/Zr filler | 37.0 | 40.0 | 42.0 |
| R812S fumed silica | 0.5 | 0.6 | 0.6 |

### Redox Paste A/B Example Preparation:

Paste A and Paste B were mixed on a paper mixing pad at an A/B weight ratio of 1.5 using a metal dental mixing spatula for 20 seconds. The mixture was then stored in a 37 °C oven and the paste curing was check by pressing against the paste with a metal spatula to see when the mixture cured into hard material. The curing time was recorded as the time that passed from mixing paste A and paste B together to the time the paste had hardened (i.e., the set time). Typically, 0.45 gram of paste A and 0.3 gram of paste B were used to do the self- curing testing.

**TABLE 11. Redox Paste A/B (1.5:1) Curing Set Times**

| EXAMPLE | PASTE A | PASTE B | SET TIME (min:second) |
|---|---|---|---|
| EX-9 | EX-A1 | EX-B1 | 4:40 |
| EX-10 | EX-A2 | EX-B1 | 4:15 |
| EX-11 | EX-A3 | EX-B1 | 3:10 |
| EX-12 | EX-A4 | EX-B1 | 5:00 |
| EX-13 | EX-A1 | EX-B2 | 3:10 |
| EX-14 | EX-A2 | EX-B2 | 2:40 |
| EX-15 | EX-A3 | EX-B2 | 4:30 |
| EX-16 | EX-A4 | EX-B2 | 3:20 |
| EX-17 | EX-A5 | EX-B1 | 4:00 |
| EX-18 | EX-A6 | EX-B1 | 4:20 |
| EX-19 | EX-A7 | EX-B1 | 4:40 |
| EX-20 | EX-A8 | EX-B1 | 2:30 |
| EX-21 | EX-A5 | EX-B2 | 2:35 |
| EX-22 | EX-A6 | EX-B2 | 2:25 |
| EX-23 | EX-A7 | EX-B2 | 2:40 |
| EX-24 | EX-A8 | EX-B2 | 2:10 |
| EX-25 | EX-A9 | EX-B1 | 3:30 |
| EX-26 | EX-A 10 | EX-B1 | 4:00 |
| EX-27 | EX-A9 | EX-B2 | 4:00 |
| EX-28 | EX-A 10 | EX-B2 | 3:00 |
| EX-29 | EX-A 11 | EX-B2 | 2:30 |
| EX-30 | EX-A6 | EX-B3 | 3:50 |

**TABLE 12. Redox Paste A/B Mechanical Properties**

| MECHANICAL STRENGTH | CE-4* | EX-30 |
|---|---|---|
| DIAMETRAL TENSILE STRENGTH, MPa +/- stdev | 21.9 +/- 0.36 | 27.2 +/- 2.5 |
| COMPRESSIVE STRENGTH, MPa +/- stdev | 123.3 +/- 8.8 | 148.7 +/- 5.4 |

| | | |
|---|---|---|
| *Comparative cement product for mechanical testing obtained under the trade designation RELYX LUTING PLUS CEMENT PRODUCT from 3M Co., Maplewood, MN. | | |

## Claims

1. An addition-fragmentation agent of the formula: 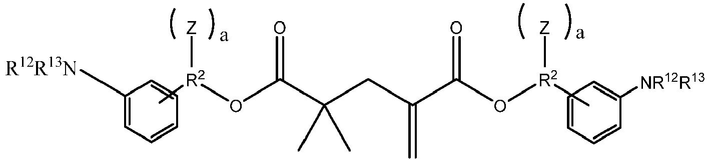 wherein
R² is a (hetero)hydocarbyl linking group of valence a+2 Z is an ethylenically unsaturated polymerizable group;
subscript a is 0 or 1
R¹² and R¹³ are independently selected from alkyl, aryl, alkaryl and aralkyl, wherein alkyl includes straight-chained, branched, cycloalkyl groups and wherein both unsubstituted and substituted groups are included.
R¹² and R¹³ may be taken together to form a heterocyclic ring,
with the proviso that the group has at least one abstractable H atom alpha to the N atom.

2. A polymerizable composition comprising the addition-fragmentation agent of claim 1, at least one free-radically polymerizable monomer, and a Type II photoinitiator.

3. The polymerizable composition of claim 2 comprising:
a) 85 to 100 parts by weight of an (meth)acrylic acid ester;
b) 0 to 15 parts by weight of an acid functional ethylenically unsaturated monomer;
c) 0 to 10 parts by weight of a non-acid functional, ethylenically unsaturated polar monomer;
d) 0 to 5 parts vinyl monomer; and
e) 0 to 5 parts of a multifunctional (meth)acrylate;
based on 100 parts by weight total monomer a) to e), and
f) 0.1 to 12 parts by weight of the addition-fragmentation agent, based on 100 parts by weight of a) to e), and
g) an initiator.

4. The polymerizable composition of claim 3 further comprising 0.01 to 5 parts of a multifunctional (meth)acrylate.

5. The polymerizable composition of claim 2 wherein the photoinitiator is a Norrish Type II photoinitiator (hydrogen abstraction).

6. The polymerizable composition of claim 2 wherein the photoinitiator is selected from camphorquinone, benzophenone, anthraquinone, 9-fluorenone, anthrone, xanthone, thioxanthone, acridone, dibenzosuberone, chromone, flavone, benzyl, and acetophenone compounds benzophenone, 4-(3-sulfopropyloxy)benzophenone sodium salt, Michler's ketone, anthraquinone, 5,12-naphthacenequinone, aceanthracenequinone, benz(A)anthracene-7,12-dione, 1,4-chrysenequinone, 6,13-pentacenequinone, 5,7,12,14-pentacenetetrone, 9-fluorenone, anthrone, xanthone, thioxanthone, 2-(3-sulfopropyloxy)thioxanthen-9-one, acridone, dibenzosuberone, acetophenone, and chromone.

7. The polymerizable composition of claim 2 wherein the free-radically polymerizable monomer is a dental resin.

8. A curable composition comprising the addition-fragmentation agents of claim 1, a polymerizable monomer, oligomer or resin, a transition metal that participates in a redox cycle and a peroxy catalyst.

9. The curable composition of claim 8 wherein the polymerizable monomer, oligomer or resin, is a dental resin.

10. The curable composition of claim 8 wherein the dental resin is an isocyanurate resin, a tricyclodecane resin, cyclic allylic sulfide resins; methylene dithiepane silane resins; and poly(meth)acryloyl-containing resins, or mixtures thereof.

11. The curable composition of any of claims 9 or 10 wherein the dental resin further comprises at least one other (meth)acrylate monomer is selected from ethoxylated bisphenol A dimethacrylate, 2-hydroxyethyl methacrylate, bisphenol A diglycidyl dimethacrylate, urethane dimethacrylate, triethlyene glycol dimethacrylate, glycerol dimethacrylate , ethylenegylcol dimethacrylate, neopentylglycol dimethacrylate (NPGDMA), polyethyleneglycol dimethacrylate, and mixtures thereof.

12. The curable composition of any of claim 9-11 wherein the peroxy catalyst is selected from persulfuric acid and salts thereof, benzoyl peroxides, hydroperoxides such as cumyl hydroperoxide, t-butyl hydroperoxide, amyl hydroperoxide, cobalt (III) chloride and ferric chloride, cerium (IV) sulfate, perboric acid and salts thereof, permanganic acid and salts thereof, perphosphoric acid and salts thereof, and mixtures thereof.

13. The composition of any of claims 8-12 further comprising a particulate additive.

## Patentansprüche

1. Ein Additions-Fragmentierungsmittel der Formel: 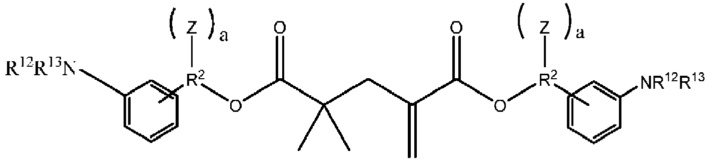 wobei
R² eine (hetero)hydocarbyl-verknüpfende Gruppe von Valenz a+2 ist
Z eine ethylenisch ungesättigte polymerisierbare Gruppe ist;
Subskript a 0 oder 1 ist
R¹² und R¹³ unabhängig ausgewählt sind aus Alkyl, Aryl, Alkaryl und Aralkyl, wobei Alkyl geradkettige, verzweigte Cycloalkylgruppen einschließt und wobei sowohl unsubstituierte als auch substituierte Gruppen eingeschlossen sind.
Wobei R¹² und R¹³ zusammen genommen werden können, um einen heterocyclischen Ring zu bilden, mit der Maßgabe, dass die Gruppe mindestens ein abspaltbares H-Atom-alpha zu dem N-Atom aufweist.

2. Eine polymerisierbare Zusammensetzung, umfassend das Additions-Fragmentierungsmittel nach Anspruch 1, mindestens ein radikalisch polymerisierbares Monomer und einen Typ-II-Photoinitiator.

3. Die polymerisierbare Zusammensetzung nach Anspruch 2, umfassend:
a) 85 bis 100 Gewichtsteile von einem (Meth)acrylsäureester;
b) 0 bis 15 Gewichtsteile von einem säurefunktionellen ethylenisch ungesättigten Monomer;
c) 0 bis 10 Gewichtsteile von einem nicht-säurefunktionellen ethylenisch ungesättigten polaren Monomer;
d) 0 bis 5 Teile Vinylmonomer; und
e) 0 bis 5 Teile von einem multifunktionellen (Meth)acrylat;
basierend auf 100 Gewichtsteilen Gesamtmonomer a) bis e), und
f) 0,1 bis 12 Gewichtsteile des Additions-Fragmentierungsmittels, bezogen auf 100 Gewichtsteile von a) bis e), und
g) einen Initiator.

4. Die polymerisierbare Zusammensetzung nach Anspruch 3, ferner umfassend zu 0,01 bis 5 Teilen ein multifunktionelles (Meth)acrylat.

5. Die polymerisierbare Zusammensetzung nach Anspruch 2, wobei der Photoinitiator ein Norrish-Typ-II-Photoinitiator (Wasserstoff-Abstraktion) ist.

6. Die polymerisierbare Zusammensetzung nach Anspruch 2, wobei der Photoinitiator ausgewählt ist aus Campherchinon, Benzophenon, Anthrachinon, 9-Fluorenon, Anthron, Xanthon, Thioxanthon, Acridon, Dibenzosuberon, Chromon, Flavon, Benzyl und Acetophenon-Verbindungen-Benzophenon, 4-(3-Sulfopropyloxy)benzophenon-Natriumsalz, Michlers-Keton, Anthrachinon, 5,12-Naphthochinon, Acacanthrenchinon, Benz(A)anthracen-7,12-dion, 1,4-Chrysenchinon, 6,13-Pentacenchinon, 5,7,12,14-Pentacentetraon, 9-Fluorenon, Anthron, Xanthon, Thioxanthon, 2-(3-Sulfopropyloxy)thioxanthen-9-on, Acridon, Dibenzosuberon, Acetophenon und Chromon.

7. Die polymerisierbare Zusammensetzung nach Anspruch 2, wobei das radikalisch polymerisierbare Monomer ein Dentalharz ist.

8. Eine härtbare Zusammensetzung, umfassend die Additions-Fragmentierungsmittel nach Anspruch 1, ein polymerisierbares Monomer, Oligomer oder Harz, ein Übergangsmetall, das an einem Redoxzyklus und einem Peroxykatalysator beteiligt ist.

9. Die härtbare Zusammensetzung nach Anspruch 8, wobei das polymerisierbare Monomer, das Oligomer oder das Harz ein Dentalharz ist.

10. Die härtbare Zusammensetzung nach Anspruch 8, wobei das Dentalharz ein Isocyanuratharz, ein Tricyclodecananharz, cyclische Allylsulfidharze; Methylendithiepansilanharze; und poly(meth)acryloyl-enthaltende Harze oder Mischungen davon ist.

11. Die härtbare Zusammensetzung nach einem der Ansprüche 9 oder 10, wobei das Dentalharz ferner mindestens ein anderes (Meth)acrylatmonomer umfasst, das ausgewählt ist aus ethoxyliertem Bisphenol-A-dimethacrylat, 2-Hydroxyethylmethacrylat, Bisphenol-A-diglycidyldimethacrylat, Urethandimethacrylat, Triethylenglycoldimethacrylat, Glyceroldimethacrylat, Ethylenglycoldimethacrylat, Neopentylglycoldimethacrylat (NPGDMA), Polyethylenglycoldimethacrylat und Mischungen davon.

12. Die härtbare Zusammensetzung nach einem der Ansprüche 9 bis 11, wobei der Peroxykatalysator ausgewählt ist aus Perschwefelsäure und Salzen davon, Benzoylperoxiden, Hydroperoxiden wie Cumylhydroperoxid, t-Butylhydroperoxid, Amylhydroperoxid, Cobalt-(III)-chlorid und Eisenchlorid, Cer-(IV)-sulfat, Perborsäure und Salzen davon, Permangansäure und Salzen davon, Perphosphorsäure und Salzen davon und Mischungen davon.

13. Die Zusammensetzung nach einem der Ansprüche 8 bis 12, ferner umfassend ein Teilchenadditiv.

## Revendications

1. Agent de fragmentation par addition de formule : 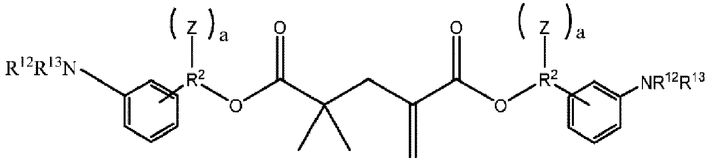 dans lequel
R2 est un groupe de liaison (hétéro)hydocarbyle de valence a+2
Z est un groupe polymérisable à insaturation éthylénique ;
l'indice a vaut 0 ou 1
R12 et R13 sont indépendamment choisis parmi alkyle, aryle, alkaryle et aralkyle, alkyle comportant des groupes à chaîne droite ramifiés, cycloalkyle, et tant des groupes non substitués que substitués étant inclus.
R12 et R13 peuvent être pris ensemble pour former un noyau hétérocyclique, à condition que le groupe ait au moins un atome de H pouvant être soustrait, en alpha par rapport à l'atome de N.

2. Composition polymérisable comprenant l'agent de fragmentation par addition selon la revendication 1, au moins un monomère polymérisable par radicaux libres, et un photoinitiateur de Type II.

3. Composition polymérisable selon la revendication 2, comprenant :
a) 85 à 100 parties en poids d'un ester d'acide (méth)acrylique ;
b) 0 à 15 parties en poids d'un monomère à insaturation éthylénique à fonction acide ;
c) 0 à 10 parties en poids d'un monomère polaire à insaturation éthylénique, sans fonction acide ;
d) 0 à 5 parties de monomère vinylique ; et
e) 0 à 5 parties d'un (méth)acrylate multifonctionnel ;
en fonction de 100 parties en poids de monomère total a) à e), et
f) 0,1 à 12 parties en poids de l'agent de fragmentation par addition, en fonction de 100 parties en poids de a) à e), et
g) un initiateur.

4. Composition polymérisable selon la revendication 3, comprenant en outre 0,01 à 5 parties d'un (méth)acrylate multifonctionnel.

5. Composition polymérisable selon la revendication 2 dans laquelle le photoinitiateur est un photoinitiateur de Norrish de Type II (retrait d'hydrogène).

6. Composition polymérisable selon la revendication 2 dans laquelle le photoinitiateur est choisi parmi camphorquinone, benzophénone, anthraquinone, 9-fluorénone, anthrone, xanthone, thioxanthone, acridone, dibenzosubérone, chromone, flavone, benzyle, et composés d'acétophénone benzophénone, sel de sodium de 4-(3-sulfopropyloxy)benzophénone, cétone de Michler, anthraquinone, 5,12-naphthacènequinone, acéanthracènequinone, benz(A)anthracène-7,12-dione, 1,4-chrysènequinone, 6,13-pentacènequinone, 5,7,12,14-pentacènetétrone, 9-fluorénone, anthrone, xanthone, thioxanthone, 2-(3-sulfopropyloxy)thioxanthén-9-one, acridone, dibenzosubérone, acétophénone et chromone.

7. Composition polymérisable selon la revendication 2 dans laquelle le monomère polymérisable par radicaux libres est une résine dentaire.

8. Composition durcissable comprenant les agents de fragmentation par addition selon la revendication 1, un monomère, oligomère ou résine polymérisable, un métal de transition qui participe à un cycle redox et un catalyseur peroxy.

9. Composition durcissable selon la revendication 8 dans laquelle le monomère, oligomère ou résine polymérisable, est une résine dentaire.

10. Composition durcissable selon la revendication 8 dans laquelle la résine dentaire est une résine isocyanurate, une résine tricyclodécane, des résines sulfure allylique cyclique ; des résines méthylène dithiépane silane ; et des résines contenant du poly(méth)acryloyle, ou des mélanges de celles-ci.

11. Composition durcissable selon l'une quelconque des revendications 9 ou 10 dans laquelle la résine dentaire comprend en outre au moins un autre monomère (méth)acrylate qui est choisi parmi diméthacrylate de bisphénol A éthoxylé, méthacrylate de 2-hydroxyéthyle, diglycidyl-diméthacrylate de bisphénol A, diméthacrylate d'uréthane, diméthacrylate de triéthylène glycol, diméthacrylate de glycérol, diméthacrylate d'éthylène glycol, diméthacrylate de néopentylglycol (NPGDMA), diméthacrylate de polyéthylène glycol, et mélanges de ceux-ci.

12. Composition durcissable selon l'une quelconque des revendications 9 à 11 dans laquelle le catalyseur peroxy est choisi parmi acide persulfurique et sels de celui-ci, peroxydes de benzoyle, hydroperoxydes tels qu'hydroperoxyde de cumyle, hydroperoxyde de t-butyle, hydroperoxyde d'amyle, chlorure de cobalt (III) et chlorure ferrique, sulfate de cérium (IV), acide perborique et sels de celui-ci, acide permanganique et sels de celui-ci, acide perphosphorique et sels de celui-ci, et mélanges de ceux-ci.

13. Composition selon l'une quelconque des revendications 8 à 12 comprenant en outre un additif particulaire.
